# EUROPÄISCHE PATENTSCHRIFT

(11) **EP 1 820 793 B1**
(45) Veröffentlichungstag und Bekanntmachung des Hinweises auf die Patenterteilung: **14.09.2011**
(21) Anmeldenummer: 07010591.1
(22) Anmeldetag: 22.05.2001
(51) Int. Cl.: C07C 211/62, C07C 211/63, C07C 211/64, C07F 9/54, C11D 3/00, C12N 15/10

(54) **Verfahren zur Stabilisierung von Nukeinsäuren in biologischen Materialien**
Process of stabilization of nucleic acids in biological materials
Procédé de stabilisation des acides nucléiques dans des matériaux biologiques

(30) Priorität: 27.06.2000 DE 10031236
(43) Veröffentlichungstag der Anmeldung: 22.08.2007
(62) Teilanmeldung aus: 01947306.5
(73) Patentinhaber: QIAGEN GmbH, 40724 Hilden (DE)
(72) Erfinder: Holländer, Vera, 59423 Unna (DE); Wyrich, Ralph, 41516 Grevenbroich (DE); Oelmüller, Uwe, 40699 Erkrath (DE)
(74) Vertreter: Leidescher, Thomas

(56) Entgegenhaltungen:
- EP-A- 0 606 712
- GB-A- 1 289 426
- US-A- 5 275 708
- US-A- 5 300 635
- US-A- 5 891 921

## Beschreibung

Die vorliegende Erfindung betrifft neue Verfahren zur Stabilisierung von Nukleinsäuren in Materialien biologischer Herkunft. Gemäß den erfindungsgemäßen Verfahren wird eine Nukleinsäure-haltige biologischen Probe mit einer wässrigen Stabilisierungslösung, die als wesentlichen Bestandteil eine kationische Verbindung der allgemeinen Formel

Y⁺R₁R₂R₃R₄X⁻

umfasst, worin
Y Stickstoff oder Phosphor
R₁, R₂, R₃ und R₄ unabhängig voneinander einen unverzweigten oder verzweigten C₁-C₂₀-Alkylrest und/oder einen C₆-C₂₀-Arylrest sowie einen C₆-C₂₆-Aralkylrest und
X⁻ ein Anion einer anorganischen oder organischen, ein- oder mehrbasischen Säure
bedeuten können
und mit mindestens einem Protonendonor als Additiv in Kontakt gebracht.

Bevorzugt sind Verfahren, in denen kationische Verbindungen eingesetzt werden, die aus einem Ammoniumsalz bestehen, in dem R₁ einen höheren Alkylrest - vorzugsweise mit 12, 14 oder 16 Kohlenstoffatomen und R₂, R₃ und R₄ jeweils eine Methylgruppe bedeutet.

Bevorzugt sind weiterhin Verfahren, in denen kationische Verbindungen eingesetzt werden, in denen R₁ eine Aralkylgruppe - vorzugsweise eine Benzylgruppen-, R₂ einen höheren Alkylrest - vorzugsweise mit 12, 14 oder 16 Kohlenstoffatomen - und R₃ und R₄ eine Methylgruppe bedeutet.

Als Anionen werden Bromid, Chlorid, Phosphat, Sulfat, Formiat, Acetat, Propionat, Oxalat oder Succinat bevorzugt.

C₁-C₆-Alkyl steht im allgemeinen für einen verzweigten oder unverzweigten Kohlenwasserstoffrest mit 1 bis 6 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
Methyl, Ethyl, Propyl, 1-Methylethyl (iso-Propyl), Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Höherer Alkylrest steht für einen verzweigten oder unverzweigten C₇-C₂₀-Alkylrest der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
verzweigtes oder unverzweigtes Heptyl, Octyl, Nonyl, Decyl, Undecyl, Dodecyl, Tetradecyl, Hexadecyl, Dodecadecyl und Eicosyl.

C₃-C₆-Alkenyl steht im Allgemeinen für einen verzweigten oder unverzweigten Kohlenwasserstoffrest mit 3 bis 6 Kohlenstoffatom(en), mit einer oder ggf. mehreren Doppelbindungen, der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-Butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl.

C₃-C₆-Alkinyl steht im Allgemeinen für einen verzweigten oder unverzweigten Kohlenwasserstoffrest mit 3 bis 6 Kohlenstoffatom(en), mit einer oder ggf. mehreren Dreifachbindungen, der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

2-Propinyl (Propargyl), 2-Butinyl, 3-Butinyl, 1-Methyl-2-propinyl, 2-Methyl-2-propinyl, 2-Pentinyl, 3-pentinyl, 4-Pentinyl, 1-Methyl-2-butinyl, 2-Methyl-2-butinyl, 3-Methyl-2-butinyl, 1-Methyl-3-butinyl, 2-Methyl-3-butinyl, 3-Methyl-3-butinyl, 1,1-Dimethyl-2-propinyl, 1,2-Dimethyl-2-propinyl, 1-Ethyl-2-propinyl, 2-Hexinyl, 3-Hexinyl, 4-Hexinyl, 5-Hexinyl, 1-Methyl-2-pentinyl, 2-Methyl-2-pentinyl, 3-Methyl-2-pentinyl, 4-Methyl-2-pentinyl, 1-Methyl-3-pentinyl, 2-Methyl-3-pentinyl, 3-Methyl-3-pentinyl, 4-Methyl-3-pentinyl, 1-Methyl-4-pentinyl, 3-Methyl-4-pentinyl, 4-Methyl-4-pentinyl, 1,1-Dimethyl-2-butinyl, 1,1-Dimethyl-2-butinyl, 1,1-Dimethyl-3-butinyl, 1,2-Dimethyl-2-butinyl, 1,2-Dimethyl-3-butinyl, 1,3-Dimethyl-2-butinyl, 1,3-Dimethyl-3-butinyl, 2,2-Dimethyl-3-butinyl, 2,3-Dimethyl-2-butinyl, 2,3-Dimethyl-3-butinyl, 1-Ethyl-2-butinyl, 1-Ethyl-3-butinyl, 2-Ethyl-1-butinyl, 2-Ethyl-2-butinyl, 2-Ethyl-3-butinyl, 1,1,2-Trimethyl-2-propinyl, 1-Ethyl-1-methyl-2-propinyl und 1-Ethyl-2-methyl-2-propinyl.

Aryl steht - steht sofern nicht anders definiert - für einen aromatischen ein- oder mehrkernigen Rest mit 4 bis 22 C-Atomen, der ggf. ein oder zweit Heteroatome enthalten kann. Als Beispiele seien genannt: Phenyl, Naphthyl, Anthracyl bzw. Pyrol, Furan, Thiophen, Pyridin, Pyridazin, Pyrimidin oder Pyrazin, und der ggf. durch Halogen (F, Cl, Br, J) - vorzugsweise Fluor - oder durch eine Alkylgruppe unabhängig voneinander ein- oder mehrfach substituiert sein kann.

Aralkyl bedeutet einen ein oder mehrkernigen Arylrest im Sinne der vorstehenden Definition der über eine C₁-C₆-Alkylen-, C₃-C₆-Alkenylen- oder eine C₃-C₆-Alkinylenbrücke, für welche die Definition der C₁-C₆-Alkyl-, C₃-C₆-Akenyl und C₃-C₆-Alkinylgruppen entsprechend gelten, an die kationische Partialstruktur gebunden ist. Im Sinne der vorliegenden Erfindung wird die Benzylgruppe bevorzugt.

Als Gegenionen X⁻ eignen sich bevorzugt alle Anionen von Halogenwasserstoffsäuren oder Anionen ein- oder zweibasischer organischer Säuren wie Acetat oder Oxalat, Malonat, Succinat oder Citrat.

Als Protonendonoren im Sinne der vorliegenden Erfindung sind in erster Linie gesättigte aliphatische Monocarbonsäuren, ungesättigte Alkenyl-carbonsäuren, gesättigte und/oder ungesättigte aliphatische C₂-C₆-Dicarbonsäuren, aliphatische Ketocarbonsäuren oder Ketodicarbonsäuren sowie Aminosäuren neben Mineralsäuren oder deren Salze allein oder in Kombination geeignet. Dabei können alle genannten organischen Säuren in unsubstituierter Form oder als substituierte Derivate eingesetzt werden, worunter - sofern nicht anders angegeben - die unsubstituierten oder ein bzw. mehrfach durch Hydroxyl-Gruppen substituierten Derivate bevorzugt werden.

Als gesättigte aliphatische Monocarbonsäuren im Sinne der vorliegenden Erfindung werden neben Ameisensäure vorzugsweise C₁-C₆-Alkyl-carbonsäuren verstanden, worunter Essigsäure, Propionsäure, n-Buttersäure, n-Valeriansäure, Isovaleriansäure, Ethyl-methyl-essigsäure (2-Methyl-buttersäure), 2,2-Dimethylpropionsäure (Pivalinsäure), n-Hexansäure, n-Octansäure, n-Decansäure sowie n-Dodecansäure (Laurinsäure) bevorzugt werden. Daneben können auch die sich von den genannten Säuren sich ableitenden Ketocarbonsäuren Verwendung finden.

Als ungesättigte Alkenyl-carbonsäuren im Sinne der Erfindung seien beispielsweise Acrylsäure (Propensäure), Methacrylsäure, Crotonsäure, iso-Crotonsäure sowie Vinylessigsäure genannt.

Bevorzugt im Sinne der vorliegenden Erfindung sind gesättigte aliphatische C₂-C₆-Dicarbonsäuren, wie zum Beispiel Oxalsäure, Malonsäure, Bersteinsäure, Glutarsäure oder Adipinsäure, worunter Oxalsäure und Bersteinsäure ganz besonders bevorzugt werden.

Besonders bevorzugt werden zur Lösung der erfidungsgemäßen Aufgaben aliphatische Hydroxi-di- und -tricarbonsäuren eingesetzt, worunter Tartronsäure, D-(+)-, L-(-)- oder DL-Äpfelsäure, (2R, 3R)-(+)-Weinsäure, (2S, 3S)-(-)-Weinsäure, meso-Weinsäure und Zitronensäure ganz besonders bevorzugt werden.

Zur Lösung der Aufgaben der vorliegenden Erfindung eigenen sich daneben auch ungesättigte Dicarbonsäuren wie Malein- oder Fumarsäure oder ungesättigte Tricarbonsäuren, wie zum Beispiel Aconitsäure.

Im Sinne der vorliegenden Erfindung können jedoch auch aliphatische Ketodicarbonsäuren als Additive eingesetzt werden, wie z. B. Mesoxalsäure und Oxalessigsäure, worunter Oxalessigsäure ganz besonders bevorzugt wird.

Des Weiteren können im Sinne der vorliegenden Erfindung Aminosäuren eingesetzt werden, worunter α-Aminosäuren - wie z. B. Aminoessigsäure (Glycin), α-Aminopropionsäure (Alanin), α-Amino-*iso*-valeriansäure (Valin), α-Amino-*iso-*capronsäure (Leucin) und α-Amino-β-methylvaleriansäure (Isoleucin) bevorzugt werden. Besonders bevorzugt findet dabei Glycin Verwendung.

Die genannten Protonendonoren können als Einzelsubstanzen bzw. in Form der reinen Stereoisomeren als auch in Mischungen eingesetzt werden.

Als weitere Additive können im Sinne der vorliegenden Erfindung ebenfalls Mineralsäuren und deren Salze eingesetzt werden. Bevorzugt kommen dabei deren Salze von Mineralsäuren - wie Phosphorsäure oder Schwefelsäure - mit Alkalimetallen oder deren Ammoniumsalze zur Anwendung. Besonders bevorzugt finden dabei Phosphorsäure und Ammoniumsulfat Verwendung.

Als Nukleinsäuren werden im Sinn der vorliegenden Erfindung Nukleinsäuren im breiteren Sinne verstanden, so z. B. Ribonukleinsäuren (RNA) wie auch Desoxyribonukleinsäuren (DNA) in allen Längen und Konfigurationen, die Doppelstrang, Einzelstrang, circuläre und lineare, verzweigte Nukleinsäuren usw. umfassen und alle möglichen Unterarten, wie z. B. monomere Nukleotide, Oligomere, Plasmide, virale und bakterielle DNA und RNA, sowie genomische und nichtgenomische DNA und RNA aus Tier- und Pflanzenzellen oder anderen Eukaryonten, mRNA in prozessierter und unprozessierter Form, tRNA, hn-RNA, rRNA, cDNA sowie alle anderen denkbaren Nukleinsäuren einschließen.

Als biologische Probe mit Nukleinsäuren können zellfreies Probenmaterial, Plasma, Körperflüssigkeiten, wie beispielsweise Blut, Serum, Zellen, Leukozytenfraktionen, Crusta Phlogistica, Sputum, Urin, Sperma, Faeces, Abstriche, Punktate, Gewebeproben jeder Art - wie z. B. Biopsien -, Gewebeteile und Organe, Lebensmittelproben, die freie oder gebundene Nukleinsäuren oder Nukleinsäure-haltige Zellen enthalten, Umweltproben, die freie oder gebundene Nukleinsäuren oder Nukleinsäure-haltige Zellen enthalten - wie z. B. Organismen (Ein- oder Mehrzeller; Insekten etc.), Pflanzen und Pflanzenteile, Bakterien, Viren, Hefen und andere Pilze, andere Eukaryonten und Prokaryonten etc., wie sie beispielsweise in der Europäischen Patentanmeldung Nr. 95909684.3 offenbart sind, auf die hiermit inhaltlich Bezug genommen wird, oder auch freie Nukleinsäuren verwendet werden.

Zum technologischen Hintergrund der Erfindung:
Aus dem Stand der Technik ist hinlänglich bekannt, dass die genetische Herkunft und funktionelle Aktivität einer Zelle durch Studien ihrer Nukleinsäuren bestimmt und untersucht werden kann. Die Analysen der Nukleinsäuren und Proteine ermöglichen den direkten Zugriff auf die Ursache der Aktivitäten von Zellen. Sie sind somit indirekten, konventionellen Methoden, wie z. B. dem Nachweis von Stoffwechselprodukten, potentiell überlegen. So werden molekularbioloigsche Analysen bereits in vielen Bereichen eingesetzt, z. B. in der medizinischen und klinischen Diagnostik, in der Pharmazie bei der Entwicklung und Evaluierung von Arzneimitteln, in der Lebensmittelanalytik sowie bei der Überwachung der Lebensmittelherstellung, in der Agrarwirtschaft bei der Züchtung von Nutzpflanzen und Nutztieren sowie in der Umweltanalytik und in vielen Forschungsgebieten.

Durch die Analyse der RNA, speziell der mRNA in Zellen, lassen sich die Aktivitäten von Genen direkt bestimmen. Die quantitative Analyse von Transkriptmustern (mRNA-Mustern) in Zellen durch moderne molekularbiologische Methoden, wie z. B. Echtzeit-Reverse-Transcriptase-PCR ("Real time RT PCR") oder Genexpressions-Chip-Analysen ermöglicht z. B. die Erkennung fehlerhaft exprimierter Gene, wodurch z. B. Stoffwechselkrankheiten, Infektionen oder die Entstehung von Krebs erkannt werden können. Die Analyse der DNA aus Zellen durch molekularbiologische Methoden, wie z. B. PCR, RFLP, AFLP, SNP oder Sequenzierung ermöglicht z. B. den Nachweis genetischer Defekte oder die Bestimmung des HLA-Typs sowie anderer genetischer Marker.

Die Analyse genomischer DNA und RNA wird auch zum direkten Nachweis von infektiösen Erregern, wie Viren, Bakterien usw. eingesetzt.

Unbedingte Voraussetzung für Nukleinsäureanalytik ist die sofortige Stabilisierung der Nukleinsäuren und Proteine nach Entnahme der biologischen Probe aus ihrer natürlichen Umgebung. Dies gilt für DNA und insbesondere RNA, die nach Entnahme der biologischen Probe sehr schnell abgebaut werden kann. Andererseits kann es nach der Entnahme der biologischen Probe durch Induktion z. B. von Streßgenen auch zur Synthese neuer mRNA-Moleküle kommen, wodurch das Transkriptmuster der Zellen verändert werden kann. Dadurch können nachfolgende Analysen verfälscht werden. Insbesondere im medizinischen Bereich ist die Stabilisierung von Nukleinsäuren notwendig, da hier häufig - z. B. in einer Praxis - Nukleinsäure-haltige Proben genommen werden, die erst nach längerer Lagerung und einem Transport in ein Labor weiter untersucht werden können.

In der Zwischenzeit können sich die in den Proben enthaltenen Nukleinsäuren verändern oder sogar vollständig zersetzen. Dies beeinflußt natürlich das Ergebnis später durchgeführter Tests massiv oder macht diese gänzlich unmöglich. Für solche Tests werden molekularbiologische Techniken wie z. B. Northern- sowie Southern-Blot-Analyse, PCR, RT-PCR, SunRise, LCR, branched-DNA (bDNA), SDA, DNA- und RNA-Chips und Arrays zur Genexpressions- und Mutationsanalystik, RFLP, AFLP, SNP-Analysen, cDNA-Synthesen, subtraktive Hybridisierung oder die Taqman-Technologie und weitere Echtzeitquantifizierungsverfahren eingesetzt. Auf der anderen Seite verkörpert die Verwendung von hochreiner, intakter Nukleinsäure - DNA oder RNA - ein Kriterium von fundamentaler Relevanz für die Anwendung bzw. Durchführung der oben genannten Tests. Daneben stellt die Isolierung der Nukleinsäure-haltigen Proben wie auch der Assays jeweils einen zeitaufwendigen Arbeitsschritt dar. Des Weiteren kann die Kontamination eines auf dem Gebiet der Molekularbiologie arbeitenden Untersuchungslabors - wie sie z. B. bei einer fehlerhaften Versuchsdurchführung auftreten kann - zu fehlerhaften Untersuchungsergebnissen führen.

### Zum Stand der Technik

Eine große Anzahl von Publikationen schlägt die Verwendung von Mischungen auf der Basis von Ethanol und Aceton als Fixative für die nachfolgende Isolierung von Nukleinsäure aus einer entsprechenden Probe - wie z. B. Gewebe - vor. Nach dem Studium dieser Literatur wird allerdings deutlich, dass derartige Ethanol/Aceton-Mischungen längst nicht alle Anforderungen, die an eine sichere RNA-Gewinnung gestellt werden, erfüllen können. So sind derartige Mischungen nicht in der Lage, die RNA vor dem Abbau zu schützen. Daneben wird nicht der Schutz der RNA in festen, aus umfangreicheren Zellverbänden aufgebauten Proben sichergestellt. Daneben sind die vorgeschlagenen Gemische leicht entzündlich bzw. explosionsgefährlich, was mit einem nicht unerheblichen Gefahrenmoment bei der Arbeit im Laboratorium verbunden ist.

Daneben befaßt sich ein mehr peripher relevanter Stand der Technik mit der Gewinnung von RNA aus fixierten bzw. konservierten Gewebeproben. Diese Abhandlungen haben im wesentlich die Eignung von histologischen Präparaten zum Gegenstand, um die Signalstärke, die bei einer *in situ* Hybridisierung erreicht wird, zu maximieren. Mit anderen Worten: derartige Experimente dienen eher dazu, RNA nachzuweisen, anstatt sie zu konservieren [US-Patente 5 196 182 und 5 260 048]. Andere Berichte haben die Gewinnung von fragmentierter RNA oder DNA aus einem fixierten Gewebe zum Gegenstand, um die so erhaltenen Fragmente in einer - eingeschränkten - molekularen Analyse mit Hilfe der PCR unterziehen zu können. Um eine derart fragmentierte DNA bzw. RNA erhalten zu können, werden die entsprechenden Proben gewöhnlich mit Proteinase K behandelt, um die strukturgebenden Gewebekomponenten abbauen zu können; erst danach wird die RNA mit einer Guanidiniumsalz-haltigen Lösung extrahiert. Allerdings ist die auf diese Art und Weise aus fixiertem Gewebe erhaltene RNA von geringer Qualität und weist nur eine Größe von ca. 200 Basen auf. Dies ist gemäß dem Stand der Technik auf eine gewisse Anzahl von bestimmten Faktoren zurückzuführen, die u.a. den nachteiligen Einfluß von endogener sowie Vernetzungsreaktioen der DNA bzw. RNA innerhalb der intrazellulären Matrix währen der Fixierung umfassen. Basierend auf dem Umstand, dass die DNA bzw. RNA in der überwiegenden Mehrzahl der Fälle zumindest partiell abgebaut ist, kann eine so gewonnene DNA bzw. RNA nicht mehr erfolgreich in einer Northem-Analyse eingesetzt werden. Eine derartig isolierte RNA könnte höchstens noch mit gewissen Erfolgsaussichten in einer RT-PCR-Reaktion eingesetzt werden, dort aber nur zur Amplifikation relativ kleiner Fragmente.

Ferner ist dem Stand der Technik die Verwendung von Ammoniumsulfat zur Konservierung von RNA bei Temperaturen oberhalb des Gefrierpunkts zu entnehmen [WO 00/06780]. Eine derartige Komposition hat unter der Bezeichnung RNA*later* Eingang in den Stand der Technik gefunden. Allerdings sind derartige wässerige Ammoniumsulfat-Lösungen nicht dazu geeignet RNA in Blut, Plasma oder Seren zu stabilisieren. Aufgrund des Umstandes, dass die genannten Proben eine hohe Proteinkonzentration aufweisen, wird beim Kontakt mit derartigen Ammoniumsalz-Lösungen sofort ein schwerlöslicher Niederschlag gebildet [RNAlater Produktinformation der Firma Ambion, Austin, Texas (USA)].

Des Weiteren ist seit geraumer Zeit aus dem Stand der Technik bekannt, sog. kationische Verbindungen zur Isolierung von Nukleinsäuren aus biologischen Proben einzusetzen. Derartige Anwendungen werden u.a. in den US-Patenten 5,010,183 und US 5,300,635, sowie in der europäischen Patentschrift EP 0442026 beschrieben. In den genannten Dokumenten wird die biologische Probe jeweils nur im Rahmen der für eine Probenvorbereitung üblichen Inkubationszeiten, d.h. im Bereich von Minuten, mit der kationischen Verbindung inkubiert; anschließend wird die Nukleinsäure weiter aufgereinigt.

Eine Überprüfung der aus dem Stand der Technik bekannten Verbindungen hat ergeben, dass die in dem Stand der Technik genannten kationischen Verbindungen - insbesondere das in den US-Patenten offenbarte Tetradecyltrimethylammonium Oxalat - alleine keine ausreichende Stabilisierung zellulärer RNA - beispielsweise bei der längeren Lagerung von Blut - gewährleisten.

Zwar sind dem Stand der Technik Versuche bekannt beispielsweise Viren in Blut über einen Zeitraum von mehreren Tagen zu stabilisieren, doch ist diesen Befunden keinerlei Hinweis auf die Unversehrtheit der RNA zu entnehmen. So beschreiben Schmidt und MacFarlane [J. Medical Virology 47 (1995) 153] die Stabilisierung von Hepatitis C Viren in Blut mittels Catrimox-14™ für sieben Tage bei Raumtemperatur. Der Nachweis der Viren erfolgte dabei mittels RT-PCR Amplifikation eines 250 Bp langen Fragments des HCV-Genoms. Der dort offenbarte Nachweis liefert jedoch kein ausreichendes Kriterium für die Intaktheit der RNA, da nur ein kurzes Fragment amplifiziert wurde. Außerdem wurde der Versuch mit einer Probe unbestimmter Viruslast durchgeführt, so dass keine Aussagen über einen Abbau von Virus-RNA während der Lagerung gemacht werden konnten.

Daneben wird in der Internationalen Patentanmeldung WO 99/29904 die Stabilisierung von DNA in Körperflüssigkeiten unter Verwendung von EDTA, EGTA oder BAPTA in Kombination mit Guanidin Hydrochlorid, Guanidin-Thiocyanat, Lithiumchlorid, Manganchlorid, Sarkosyl, SDS, Natriumperchlorat, Natriumsalicylat und Natriumthiocyanat beschrieben. Außerdem ist dem Stand der Technik zu entnehmen, dass Phenol-haltige Reagenzien wie z. B. Trizol™ zur Stabilisierung von RNA während der Lagerung verwendet werden können. - Alle diese Reagenzien sind jedoch sehr gesundheitsschädlich und damit nicht für Routineanwendungen geeignet.

Der vorliegenden Erfindung liegt daher die Aufgabe zugrunde, ein Verfahren zur Verfügung zu stellen, welches die Stabilisierung von RNA in Gegenwart von Gewebe bzw. Blut, Plasma oder Serum erlaubt.

Der vorliegenden Erfindung liegt daneben die Aufgabe zugrunde, ein Verfahren bereit zu stellen, bei dem eine Stabilisierungslösung eingesetzt werden kann, deren Bestandteile nicht gesundheitsschädlich sind, wodurch das Verfahren z. B. auch für eine Stabilisierung von biologischem Nukleinsäure-haltigen Probenmaterial während des Transportes vom Ort der Entnahme zu einem Labor ohne Gesundheitsgefahren für das mit der Probenbearbeitung befasste Personal eingesetzt werden kann.

Eine weitere Aufgabe der vorliegenden Erfindung besteht daneben darin, ein Verfahren zur Verfügung zu stellen, das unter Anwendung einer Komposition in Form einer Stabilisierungslösung arbeitet, mit der die Voraussetzung erfüllt wird, dass auch das Stabilisierungsreagenz selbst in Lösung stabil bleibt und daher keinerlei Vorbehandlung - wie z. B. das Auflösen schwerlöslicher Präzipitate - beim Anwender erforderlich macht. Derartige Vorbehandlungen sind stets mit der Gefahr der Variation in der Stabilisierungseffizienz verbunden.

Eine weitere Aufgabe der vorliegenden Erfindung besteht darin, ein Verfahren zur Verfügung zu stellen, das vielseitig einsetzbar ist, d. h.: das bei einem breiten Spektrum biologischer Nukleinsäure-haltigen Proben angewendet werden kann.

Überraschenderweise wurde nun festgestellt, dass die Stabilisierung von Nukleinsäuren über einen längeren Zeitraum gelingt, wenn man die Nukleinsäuren einer biologischen Probe mit einer kationischen Verbindung, wie sie u.a. in den US-Patenten 5 010 183 und 5 300 635 offenbart ist, in Kontakt bringt und erfindungsgemäß mit einem oder mehreren der eingangs beschriebenen Additive versetzt. Additive, die sich bevorzugt für die Lösung der erfindungsgemäßen Aufgabe eignen, sind in Tab. 1 aufgeführt:

**Tabelle 1**

| Bezeichnung | Formel |
|---|---|
| Essigsäure | CH₃-COOH |
| Oxalsäure | HOOC-COOH |
| Malonsäure | HOOC-CH₂-COOH |
| Tartronsäure | HOOC-CHOH-COOH |
| Bernsteinsäure | HOOC-CH₂-CH₂-COOH |
| Äpfelsäure | HOOC-CHOH-CH₂-COOH |
| Weinsäure | HOOC-CHOH-CHOH-COOH |
| Glutarsäure | HOOC-CH₂-CH₂-CH₂-COOH |
| Adipinsäure | HOOC-CH₂-CH₂-CH₂-CH₂-COOH |
| Zitronensäure | HOOC-CH₂-COHCOOH-CH₂-COOH |
| Maleinsäure | HOOC-CH=CH-COOH |
| Oxalessigsäure | HOOC-CO-CH₂-COOH |
| Glycin | H₂N-CH₂-COOH |
| Ammoniumsulfat | (NH₄)₂SO₄ |
| Phosphat | H₃PO₄ K und Na Salz |

Das Additiv kann in unterschiedlichen Konzentrationen in dem Stabilisierungsreagenz vorliegen; beispielsweise kann es in Mischungen der Stabilisierungslösung mit Blut in einem Volumenverhältnis von 1:1 - bevorzugt 3:1 in einer Konzentration von 50mM bis zur Sättigung , bevorzugt 100 mM bis 1 M und besonders bevorzugt in einer Konzentration von 200 - 500 mM zugegen sein. Dabei können in Abhängigkeit von der Natur des Additivs sich andere Konzentrationsbereiche als vorteilhaft erweisen. Daneben ist auch der Einsatz von Kombinationen verschiedener Additive in dem erfindungsgemäßen Verfahren möglich.

Die kationische Verbindung weist in der wässerigen Lösung der Komposition eine Konzentration in einem Bereich zwischen 0,01 Gew.-% und Sättigung, bevorzugt zwischen 0,1 Gew.-% und Sättigung und besonders bevorzugt zwischen 0,5 Gew.-% und 15 Gew.-% und ganz besonders bevorzugt zwischen 2 Gew.-% und 10 Gew.-% auf.

Naturgemäß werden bei der Zugabe einer Lösung von kationischer Verbindung und Additiv die jeweiligen optimalen Konzentrationen durch das Volumen der biologischen Probe und das Volumenverhältnis von Stabilisierungslösung zur biologischen Probe bestimmt.

Der pH-Wert der Mischung aus kationischer Verbindung und Additiv kann in Abhängigkeit von der Probe im Allgemeinen über einen weiten pH Bereich (pH 2 bis 12) variiert werden und liegt bevorzugt in einem Intervall von pH 2 bis pH 10 und besonders bevorzugt in einem Intervall von pH 3 bis 8. Dabei ist der bevorzugte pH-Bereich abhängig von der eingesetzten biologischen Probe. - Für Blut, Plasma und Serum ist ein pH-Wert in einem Bereich zwischen pH 2 und pH 6 und besonders zwischen pH 3 und pH 4 bevorzugt.

Für biologische Proben, wie auch für andere zelluläre Körperflüssigkeiten außer Blut, Plasma und Serum, oder z. B. Bakterien, Punktate, Zellen, Gewebe und weiterer biologischer Proben - wie oben beschrieben - liegt der pH-Wert in der Stabilisierungslösung bestehend aus kationischer Verbindung und Additiv bevorzugt in einem Bereich von pH 3 bis pH 10 und besonders bevorzugt in einem Intervall von pH 4 bis pH 8.

Zur Stabilisierung von Nukleinsäuren in biologischen Proben kann die Probe mit einer Lösung, welche die kationische(n) Verbindung(en) und Additive enthält, vermischt werden. Dabei ist ein Zugabevolumen von 0,1 bis 10.000 Volumen der biologischen Probe möglich; bevorzugt wird ein Zugabevolumen in einem Bereich von 1 bis 1000 und ganz besonders bevorzugt in einem Intervall von 1 bis 100 Volumen. In Abhängigkeit von der Art der Probe - wie beispielsweise Proben aus feinen Nadelbiopsien oder Niedrigzellkulturen können jedoch u.U. auch wesentlich höhere Volumina in Frage kommen.

Ebenso können die oben genannten kationischen Verbindungen und Additive auch als Feststoff zugesetzt werden, wenn die biologische Probe selbst Flüssigkeit zur Lösung des Feststoffes enthält (wie z. B. zellhaltige Körperflüssigkeiten, Zellen in Medium, Urin) oder Flüssigkeit, z. B. Wasser, zur Lösung des Feststoffes hinzu gegeben wird. Die Zugabe als Feststoff bietet den Vorteil, dass Feststoffe meist chemisch stabiler sind und ihre Zugabe zur Probe oft einfacher durchführbar ist.

Darüber hinaus ist insbesondere bei sehr kompakten biologischen Proben, wie beispielsweise Geweben, eine Zerkleinerung bzw. Homogenisation der Probe in der Stabilisierungslösung bzw. vor Mischung mit der Stabilisierungslösung möglich, um durch z. B. mechanische, chemische, physikalische oder enzymatische Einwirkung auf die Probe die Freisetzung der Nukleinsäuren oder einzelner Zellen bzw. Zellverbände durch Zerstörung einer kompakten Probe zu unterstützen. Eine mechanische Einwirkung kann z. B. mit einem elektrischen Messer, einer Kugelmühle oder durch Pressen durch eine Spritze geschehen, während sich geeignete Enzyme zur Einwirkung auf die Probe - beispielsweise Hydrolasen, Proteasen oder Lipasen - anbieten.

Daneben kann die Probe auf rein physikalischem Wege - beispielsweise mittels Ultraschall - vorbehandelt werden.

Die Vorbehandlung kann des Weiteren auf chemischem Wege - entweder allein oder in Kombination mit rein physikalischen Methoden - erfolgen. Als Mittel zur Unterstützung der Lyse können z. B. aliphatische Alkohole - insbesondere Isopropanol - oder Aldehyde bzw. Dialdehyde - wie z. B. Glyoxal - oder auch Phenole oder Phenolderivate - wie z. B. 2-Biphenylol oder ionische, zwitterionische und nichtionische Verbindungen, - wie z. B. Sulfhydryl - oder reduzierende Reagenzien - wie z. B. Dithiothreitol und β-Mercaptoethanol - oder Phosphorsäurederivate - wie z. B. Tributylphosphat - oder aber chaotrope Reagenzien, wie z. B. Harnstoff, Guanidiniumthiocyanat oder Guanidinium-hydrochlorid - oder Salze einzeln oder in Kombination verwendet werden.

Weitere Möglichkeiten zur mechanischen, chemischen, physikalischen oder enzymatischen Einwirkung auf Proben sind dem Fachmann bekannt und sollen hier umfasst sein.

Die Lagerung des Probenmaterials kann - den jeweiligen Bedürfnissen folgend - über längere Zeiträume, wie z. B. von 1 bis zu 14 Tage oder länger, bei Raumtemperatur aber auch bei erhöhten Temperaturen, wie z. B. 40°C oder mehr, und auch bei erniedrigten Temperaturen wie z. B. 4°C oder -20°C oder weniger erfolgen.

Im Anschluß an die Lagerung der biologischen Probe in der Lösung der o. g. Verbindungen können entweder direkt Nukleinsäure-Analysetechniken angeschlossen werden, oder es kann eine Aufreinigung der Nukleinsäuren aus der Probe stattfinden.

Eine direkte Detektion/Analytik von Nukleinsäuren ist beispielsweise in Blotting-Techniken, gelelektrophoretischen Methoden zur Auftrennung von Biomolekülen und durch chromatographische Methoden zu sehen.

Zur Aufreinigung der Nukleinsäuren aus der biologischen Probe werden die freien Nukleinsäuren oder Nukleinsäure-haltige Zellen oder Partikel z. B. durch Zentrifugation oder Filtration von der restlichen Lösung abgetrennt und einer weiteren Aufreinigung zugeführt, die vorteilhaft in einem geringen Volumen stattfinden kann, wie in den US-Patenten US 5.010.183, US 5.300.635 und in der Europäischen Patentanmeldung mit der Anmeldenummer 99103457.0 beschrieben.

Die direkte Abtrennung der Nukleinsäuren bzw. der Nukleinsäure-haltigen Zellen oder Partikel im Lagerungsgefäß ermöglicht dabei die Einsparung zusätzlicher Schritte zur Überführung der Probe in andere Gefäße zur Aufreinigung und vermindert somit sowohl Probenverluste als auch die Gefahr von Verwechslungen und von Kontamination durch Verschleppungen von Nukleinsäuren von Probe zu Probe. Das erfindungsgemäße Verfahren ermöglicht unter Anwendung dieser Stabilisierungsreagenzien ein 1-Schritt-Verfahren zur Stabilisierung und direkten Isolierung von Nukleinsäuren in biologischen Proben, wobei RNA und DNA alternativ aus der biologischen Probe öder parallel aus einer Probe isoliert werden kann.

Durch die Stabilisierung von Nukleinsäuren mit Hilfe des erfindungsgemäßen Verfahrens unter Verwendung einer Stabilisierungslösung, die eine oder mehrere kationische Verbindung(en) und ein oder mehrere Additiv(e) enthält, wird erreicht, dass sich die Nukleinsäuren in einer Probe auch bei längerer Lagerung oder während eines Transports nicht verändern. Somit wird die Genauigkeit später durchgeführter Tests deutlich erhöht. In bestimmten Fällen - wenn z. B. das Probenmaterial über weite Strecken transportiert oder länger gelagert werden muss - macht das erfindungsgemäße Verfahren diese Tests nach einem derartigen Zeitraum überhaupt erst möglich.

Die Vorteile dieser Erfindung liegen insbesondere sowohl im Bereich der Forschung, z. B. für die Analyse von Transkriptspiegeln, die direkt nach der Entnahme fixiert werden müssen, als auch im Bereich klinischer Analysen - wie z. B. molekulare Diagnostik -, wo Patientenproben nach der Entnahme während Lagerung und Transport bis zur Analyse ebenfalls stabilisiert werden müssen. Insbesondere findet die Isolierung und Stabilisierung von Nukleinsäuren Anwendung in der Tumordiagnostik, in der Diagnostik erblich bedingter Krankheiten sowie in der Virusdiagnostik und dem Virus-Monitoring und der Diagnose und dem Monitoring anderer infektiöser Erreger, sowie in der Analyse von Genexpressionsmustern.

Die Anwendungsbereiche der vorliegenden Erfindung erstrecken sich dabei nicht nur auf medizinische bzw. zoologische Anwendungfelder, sondern umfassen auch die Analyse botanischer, pilzlicher und prokaryotischer Systeme. Die Stabilisierung und Isolierung von Nukleinsäuren aus Pflanzen und Pflanzenteilen, Algen, Pilzen sowie Bakterien aus Kulturen und natürlichen Habilitaten finden im Bereich der Forschung Anwendung, z. B. für die Analyse von Transkriptspiegeln und Genexpressionsmustern sowie zur Identifizierung und Quantifizierung von Spezies in komplexen Populationen, beispielsweise von Bakterien in einer Bodenprobe.

Darüber hinaus erstreckt sich das Anwendungspotential auch auf weitere analytische Bereiche wie z. B. auf die Lebensmittelanalytik.

Die vorliegende Erfindung wird anhand folgenden Beispiele sowie der Figs. erläutert. In der Beschreibung und in den Beispielen werden die folgenden Abkürzungen verwandt:
- AFLP: Längenpolymorphismus amplifizierter Fragmente
- A. dest.: Destilliertes Wasser
- BAPTA: 1,2-Bis(2-aminophenoxy)ethan-N,N,N',N'-tetraessigsäure
- EcoRI: Restriktionsenzym Escherichia coli Stamm R
- E₂₆₀/E₂₈₀: Quotient der Extinktionen bei 260 und 280 nm
- EDTA: Ethylendiamin-N,N,N',N'-tetraessigsäure
- EGTA: [Ethylenbis(oxyethylennitrilo)]tetraessigsäure
- GAPDH: Glycerinaldehyd-3-phosphat-Dehydrogenase
- Hind III: Restriktionsenzym Haemophilus influenzae
- hugl: human homologue of giant larvae
- IFN-γ: Interferon-gamma
- LM: Längenmarker
- MOPS: 3-(N-Morpholino)-2-hydroxypropansulfonsäure
- nb: nicht bestimmt
- Nonidet P40: Imbentin-N/52; Octylphenylpolyethylenglycol
- OD: optische Dichte
- PBS: phosphate buffered saline
- PCR: Polymerase Chain Reaction
- RFLP: Restriktionsfragment-Längen-Polymorphismus
- rpm: Umdrehungen pro Minute
- mRNA: messenger RNA
- rRNA: ribosomale RNA
- RT: Raumtemperaur
- RT-PCR: Reverse Transcriptase PCR
- SDS: Natriumdodecylsulfat
- SNP: Single Nucleotide Polmorphism
- SSC: Kochsalz/Natriumcitrat-Lösung
- TBE: Tris-Borat-EDTA-Puffer
- Tris: 2-Amino-2-(hydroxymethyl)-1,3-propandiol
- U: Einheiten

Nicht aufgeführte Abkürzungen - wie z.B. h für Stunde(n) - sind dem Fachmann in ihrer Bedeutung geläufig bzw. durch ihre Verwendung im Stand der Technik hinreichend bekannt.

### Erläuterungen zu den Figuren und den ihnen zugrunde liegenden Experimenten

**Fig. 1** zeigt die Stabilisierung der RNA in Blut mittels Tetradecyltrimethylammonium-Oxalat (TTAOx) in verschiedenen Carbonsäure-Puffern unterschiedlicher pH-Werte

**Fig. 2** zeigt die Stabilisierung der RNA in Vollblut mittels Tetradecyltrimethylammonium-Oxalat, gepuffert mit Weinsäure pH 3 in unterschiedlichen Konzentrationen.

**Fig. 3** zeigt die Stabilisierung der RNA in Vollblut mittels Tetradecyltrimethylammonium-Oxalat gepuffert mit 250 mM Weinsäure pH 3.

**Fig. 4** zeigt die Stabilisierung von RNA in Vollblut mittels Tetradecyltrimethylammonium-Oxalat, gepuffert mit Weinsäure pH 3,7 als Ergebnis einer Northern-Hybridisierung mit einer radioaktiv markierten Sonde für die mRNA des GAPDH-Gens (A) und des IFN-γ-Gens (B). Auch nach Lagerung über einen Zeitraum von 72 h ist in diesem Experiment die mRNA des GAPDH-Gens und des IFN-γ-Gens nachweisbar.

**Fig. 5** zeigt die Stabilisierung der genomischen DNA in Blut mittels Tetradecyltrimethylammonium-Oxalat gepuffert mit Weinsäure bei pH 3,7.

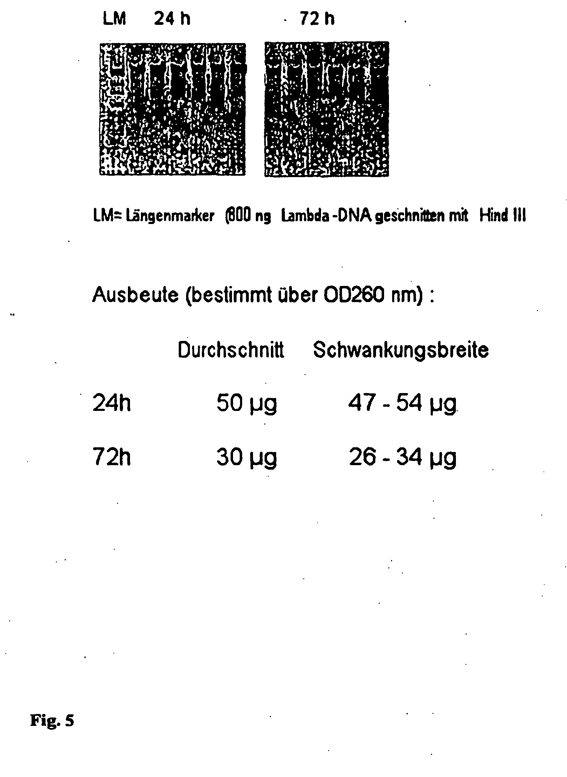

Neben der zellulären RNA kann mit dem hier entwickelten Verfahren auch die genomische DNA aus den weißen Blutkörperchen stabilisiert und anschließend durch Bindung an eine Silica-Membran isoliert werden. Fig. 5 zeigt, dass auch nach Lagerung für 72 h hochmolekulare DNA (Länge > 20 kB) isoliert wird.

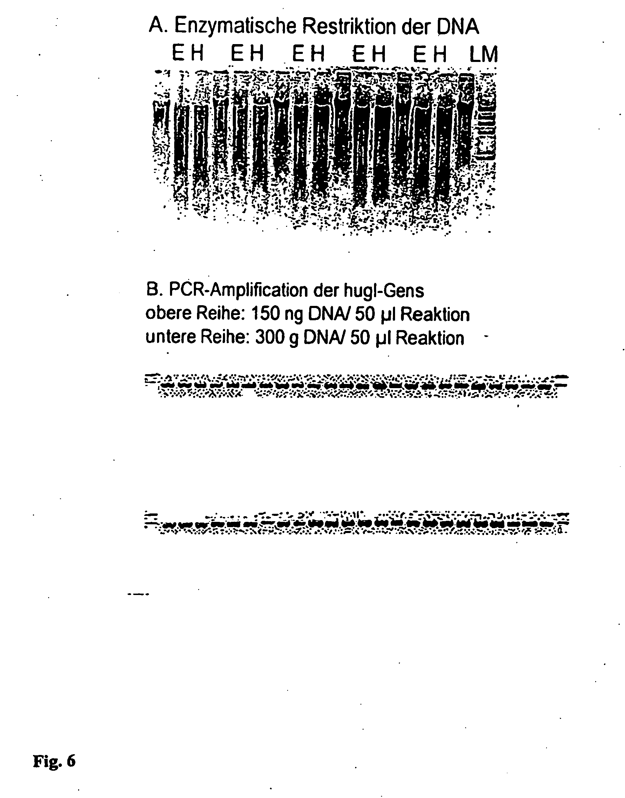

**Fig. 6** zeigt die Resultate bei der Verwendung der genomischen DNA in enzymatischen Reaktionen. Die nach Lagerung für 24 bzw. 72 Stunden isolierte DNA (siehe Beispiel 5) wird in verschiedenen enzymatischen Reaktionen eingesetzt.

A. Je 2 µg der DNA werden mit 6 U der Restriktionsenzyme EcoRl (E) bzw. Hind III (H) für 3 h bei 37°C geschnitten und anschließend auf einem 0,8 %-igen Agarose/TBE-Gel aufgetrennt. Zur Kontrolle ist jeweils die ungeschnittene DNA ausgetragen.

B. Jeweils 150 bzw. 300 ng der genomischen DNA werden in eine PCR Reaktion eingesetzt (Gesamtvolumen 50 µl), bei der ein 1,1 kB langes Fragment des hugl-Gens (human homologue of giant larvae) amplifiziert wird. Die PCR-Produkte werden auf einem 1,2 %-igen Agarose/TBE-Gel aufgetrennt.

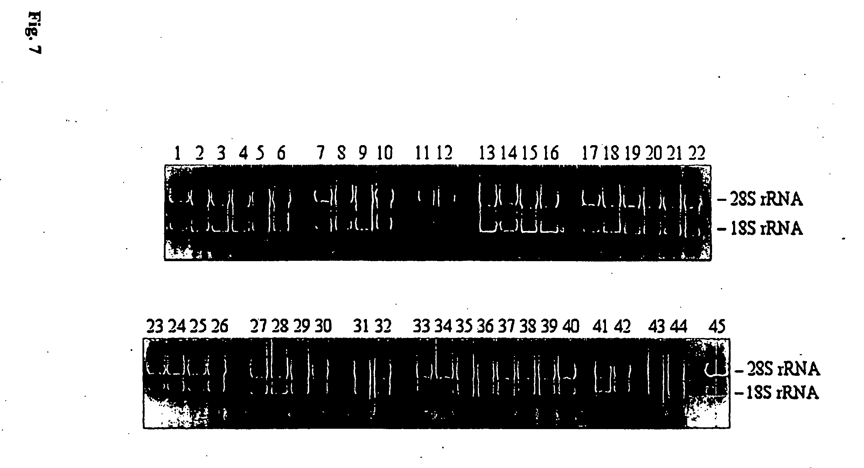

**Fig. 7** zeigt die Stabilisierung von RNA in Plasma mittels Tetradecyltrimethylammonium-Oxalat gemischt mit verschiedenen Additiven. Dabei werden alle Proben in Form von Doppelbestimmungen angesetzt: je 30 µl der Eluate werden in einem 1 % Agarose-Formaldehyd-MOPS-Gel aufgetrennt. Die jeweiligen Proben sind in Tabelle 2 beschrieben.

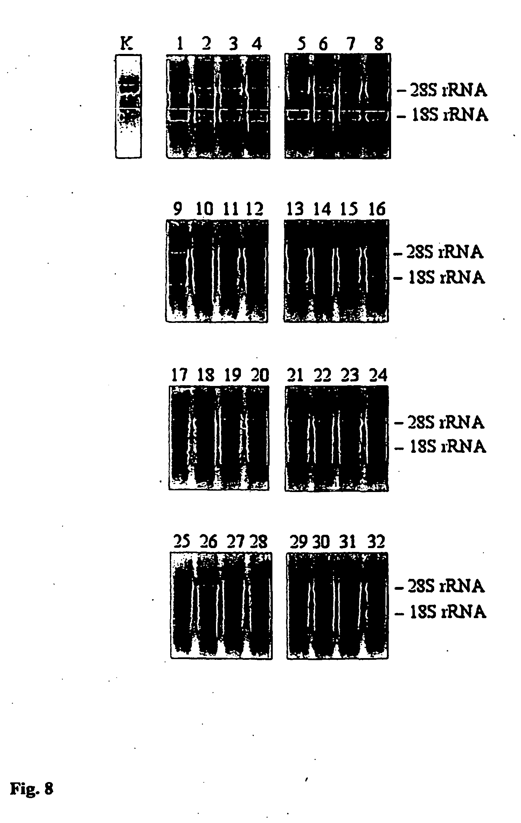

**Fig. 8** zeigt die RNA-Stabilisierung in Plasma mittels Tetradecyltrimethylammonium-Oxalat gemischt mit Wein- bzw. Tartronsäure über verschiedene Zeiträume.

Dabei werden alle Proben in Form von Doppelbestimmungen angesetzt je 30 µl der Eluate werden in einem 1% Agarose-Formaldehyd-MOPS-Gel aufgetrennt. - Die jeweiligen Proben sind in Tabelle 3 beschrieben.

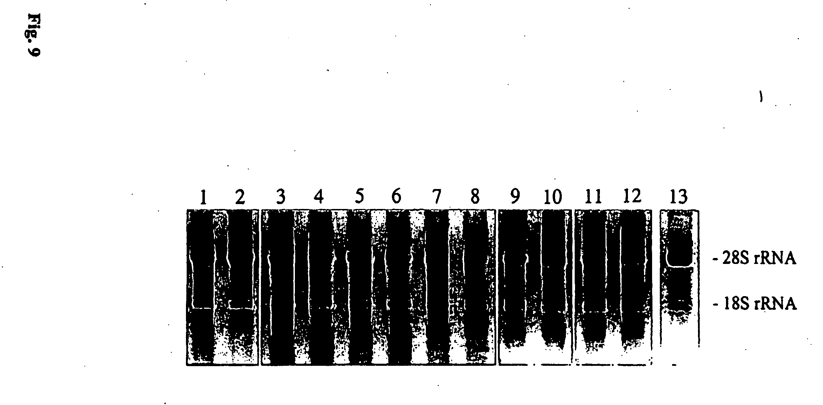

**Fig. 9** zeigt die RNA-Stabilisierung in 1 ml Plasma mittels Tetradecyltrimethylammonium-Oxalat gemischt mit verschiedenen Additiven.

Dabei werden alle Proben in Form von Doppelbestimmungen angesetzt je 30 µl der Eluate werden in einem 1% Agarose-Formaldehyd-MOPS-Gel aufgetrennt.

Die jeweiligen Proben sind in Tabelle 4 beschrieben.

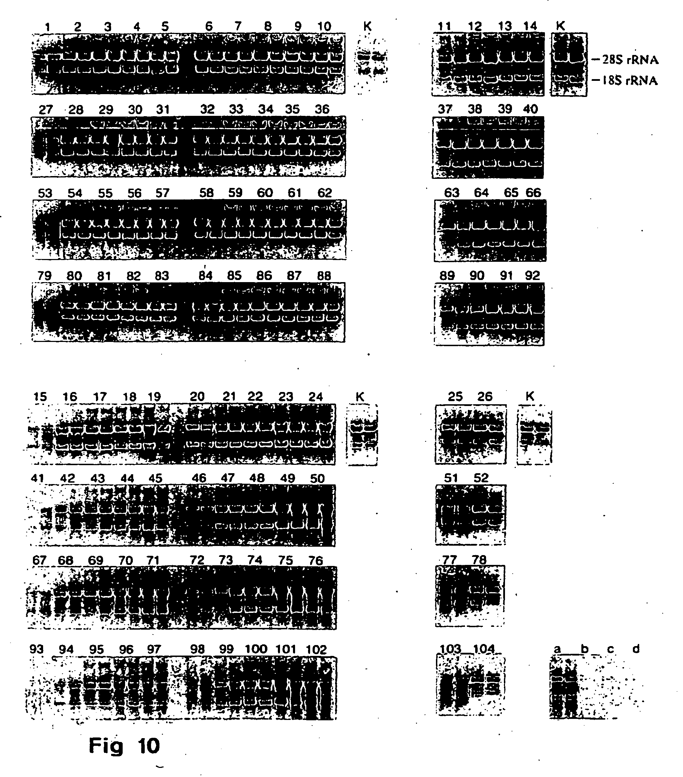

**Fig. 10** zeigt die RNA-Stabilisierung in Hela-Zellen mittels Tetradecyltrimethylammonium-Oxalat gemischt mit verschiedenen Additiven.
Dabei werden die Proben in Form von Doppelbestimmungen angesetzt, die Proben 14, 40, 66 und 92 als Einfachbestimmung je 20 µl der Eluate werden in einem 1% Agarose-Formaldehyd-MOPS-Gel aufgetrennt. Die jeweiligen Proben sind in Tabelle 5 beschrieben.

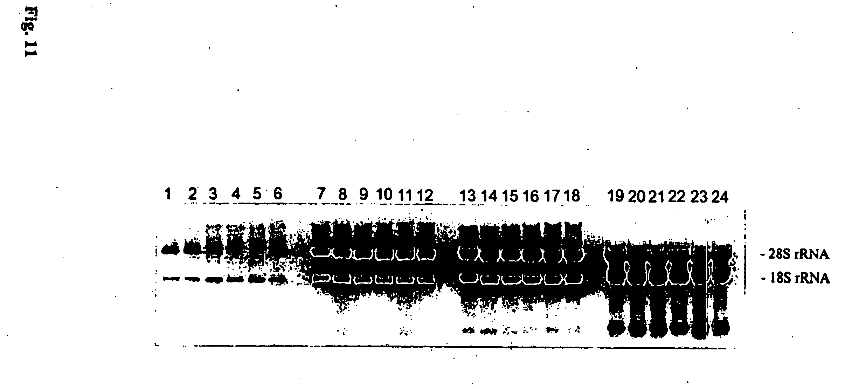

**Fig. 11** zeigt die RNA-Stabilisierung in unterschiedlichen Mengen von Hela-Zellen. Dabei werden alle Proben in Form von Doppelbestimmungen angesetzt je 20 µl der Eluate werden in einem 1% Agarose-Formaldehyd-MOPS-Gel aufgetrennt. Die jeweiligen Proben sind in Tabelle 7 beschrieben.

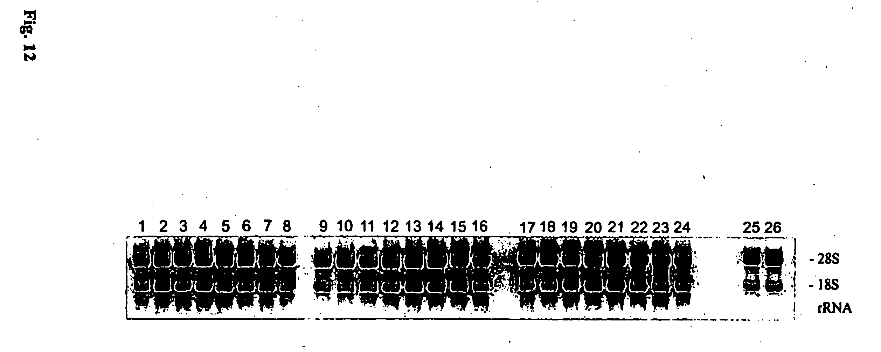

**Fig. 12** zeigt die RNA-Stabilisierung in Macrophagen. Dabei werden alle Proben in Form von Doppelbestimmungen angesetzt. Je 20 µl der Eluate werden in einem 1% Agarose-Formaldehyd-MOPS-Gel aufgetrennt. Die jeweiligen Proben sind in Tabelle 9 beschrieben.

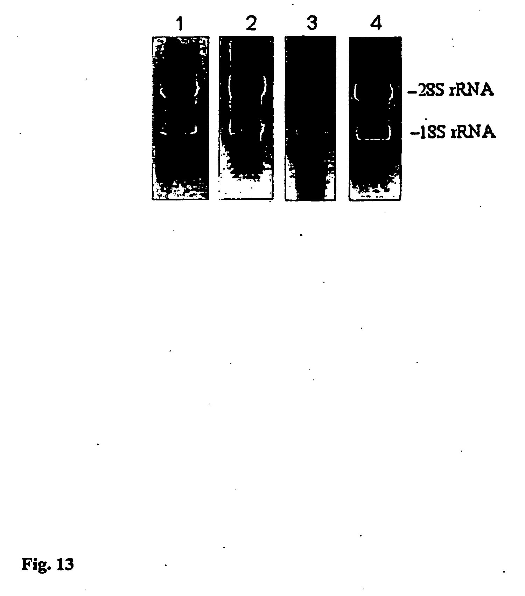

**Fig. 13** zeigt die RNA-Stabilisierung in adhärenten Hela-Zellen ohne Entfernung des Mediums. Dabei werden je 20 µl der Eluate werden in einem 1% Agarose-Formaldehyd-MOPS-Gel aufgetrennt. Die jeweiligen Proben sind in Beispiel 13 beschrieben.

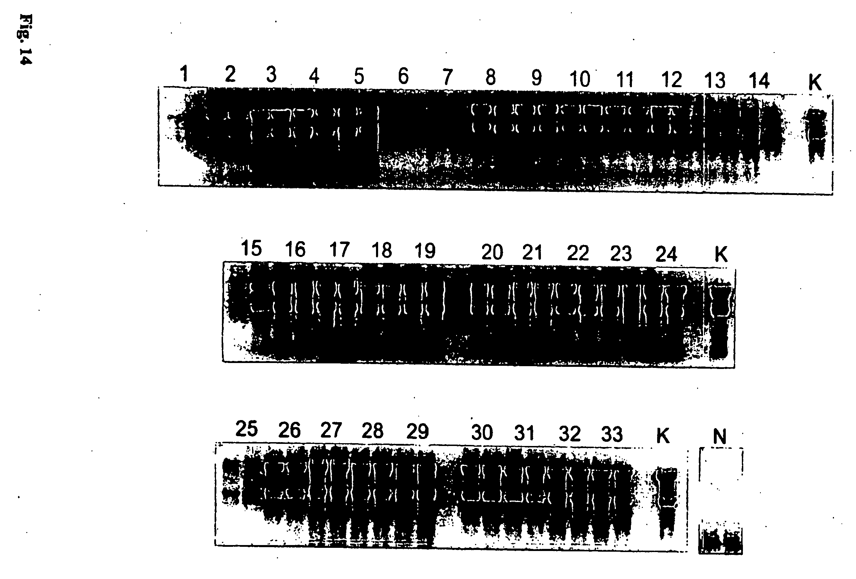

**Fig. 14** zeigt die RNA-Stabilisierung in Nierengewebe mittels TetradecyltrimethylAmmonium-Oxalat gemischt mit verschiedenen Additiven. Dabei werden alle Proben in Form von Doppelbestimmungen angesetzt; je 20 µl der Eluate werden in einem 1% Agarose-Formaldehyd-MOPS-Gel aufgetrennt. Die jeweiligen Proben sind in Tabelle 12 beschrieben.

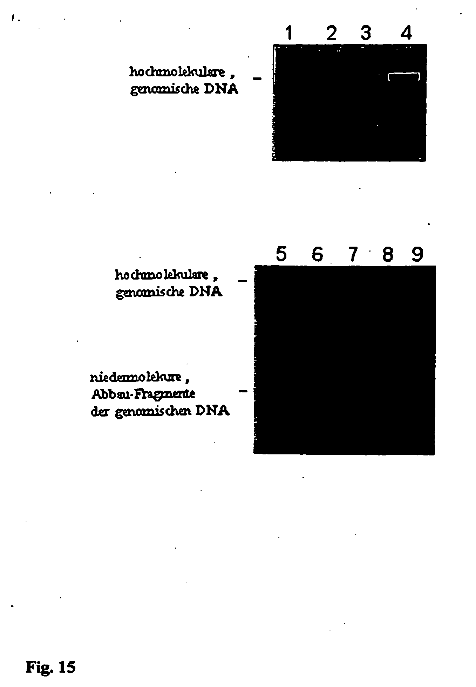

**Fig. 15** zeigt die DNA-Stabilisierung und -Isolierung parallel zur RNA-Stabilisierung und -Isolierung. Dabei werden je 40 µl der Eluate in einem 0,8% Agarose-TBE-Gel aufgetrennt. Die jeweiligen Proben sind in Beispiel 15 beschrieben.

### Beispiele

### Beispiel 1:

### Stabilisierung der RNA in Blut mittels Tetradecyltrimethylammonium-Oxalat (TTAOx) in verschiedenen Carbonsäure-Puffern mit unterschiedlichen pH-Werten

Als Additive werden Carbonsäuren verschiedener Kettenlänge ausgewählt. Außerdem werden Mono-, Di- und Tri-Carbonsäuren, hydroxylierte und nicht-hydroxylierte Carbonsäuren getestet. Alle Substanzen werden zur Stabilisierung in Kombination mit der kationischen Verbindung Tetradecyltrimethyammonium-Oxalat eingesetzt. Dabei werden sowohl der pH-Wert als auch die Konzentration der Substanzen variiert.

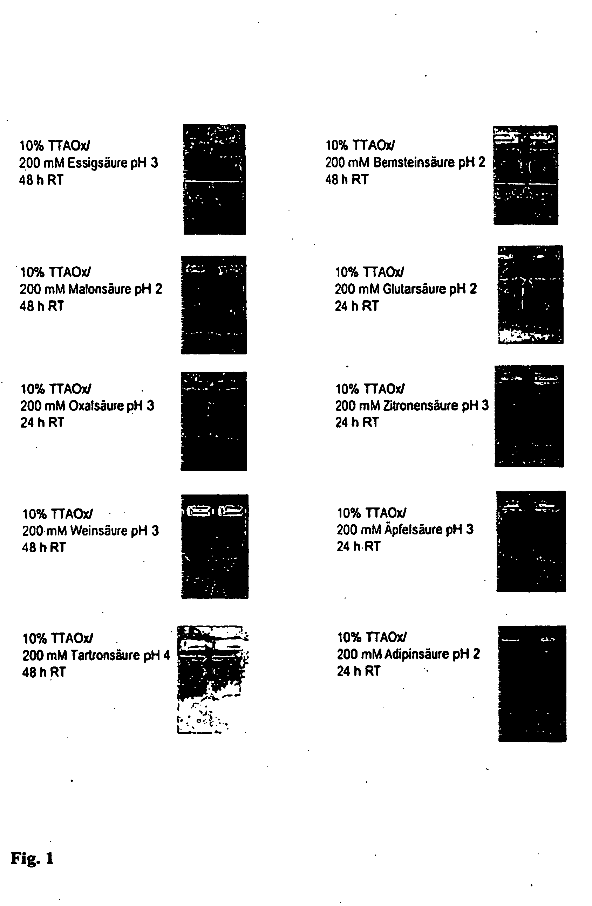

Fig. 1 zeigt die Ergebnisse der Untersuchungen. In allen Fällen kann auch nach 24 bzw. 48 h intakte RNA isoliert werden. Die z.T. geringen RNA Mengen hängen mit dem geringen Blutvolumen zusammen, das aufgearbeitet wurde und mit dem unterschiedlichen RNA Gehalt in verschiedenen Blutproben. Bei diesen Experimenten wurde ein Anteil der genomischen DNA ebenfalls in den RNA-Fraktionen erhalten.

500 µl Blut werden mit 500 µl eines Puffers, bestehend aus 10 % (w/v) Tetradecyltrimethylammonium-Oxalat - gepuffert mit unterschiedlichen Carbonsäuren jeweils in einer Konzentration von 200 mM - sowie den für die jeweilige Carbonsäure jeweils unterschiedlichen pH-Werten, für 24 und 48 Stunden bei RT gelagert. Zur Isolierung der RNA werden die Komplexe - bestehend aus kationischer Verbindung und Nukleinsäure - abzentrifugiert; das Pellet wird einmal mit Wasser gewaschen, erneut abzentrifugiert und in 300 µl eines handelsüblichen Lysepuffers - wie z. B. RLT Puffer der Firma QIAGEN - aufgenommen. Die Probe wird mit 360 µl Wasser verdünnt und für 10 Minuten bei 55°C mit 40 µl Proteinase K behandelt. Anschließend wird die Probe zentrifugiert, der Überstand mit Ethanol versetzt und auf eine Silica-Membran enthaltende Spin-Säule aufgetragen. Die Probe wird mittels Zentrifugation durch die Membran geführt. Die Spin-Säule wird einmal mit einem kommerziell erhältlichen Guanidinium-Isothiocyanat-haltigen Waschpuffer - beispielsweise mit dem Puffer RW1 der Firma QIAGEN - und zweimal mit einem handelsüblichen, alkoholhaltigen Waschpuffer - z. B. Puffer RPE der Firma QIAGEN - gewaschen, und die RNA anschließend in 60 µl RNase freiem Wasser, das ebenfalls mittels Zentrifugation durch die Membran geführt wird, eluiert. Jeweils 30 µl des Eluates werden auf einem 1,2 %-igen Agarose/Formaldehyd Gel aufgetrennt.

### Beispiel 2:

Stabilisierung der RNA in Vollblut mittels Tetradecyltrimethylammonium-Oxalat und Weinsäure (gepuffert) bei pH 3 in unterschiedlichen Konzentrationen.

500 µl Blut werden mit 500 µl eines Puffers, bestehend aus 10 % (w/v) Tetradecyltrimethylammonium-Oxalat und 50-500 mM Weinsäure pH 3 für 2,5, 24 und 48 Stunden bei RT gelagert. Die Isolierung der RNA erfolgt wie in Fig. 1 beschrieben, mit dem Unterschied, dass zusätzlich die genomische DNA durch eine DNase Behandlung der Probe mit dem "RNase-Free-DNase Set" der Firma QIAGEN entfernt wird. Die RNA wird mit 80 µl RNase freiem Wasser eluiert. Jeweils 30 µl des Eluates werden auf einem 1,2 %-igen Agarose/Formaldehyd Gel aufgetrennt.

### Beispiel 3:

Stabilisierung der RNA in Vollblut mittels Tetradecyltrimethylammonium-Oxalat gepuffert mit 250 mM Weinsäure bei pH 3.

### Bestimmung der Integrität, Ausbeute und Reinheit der RNA:

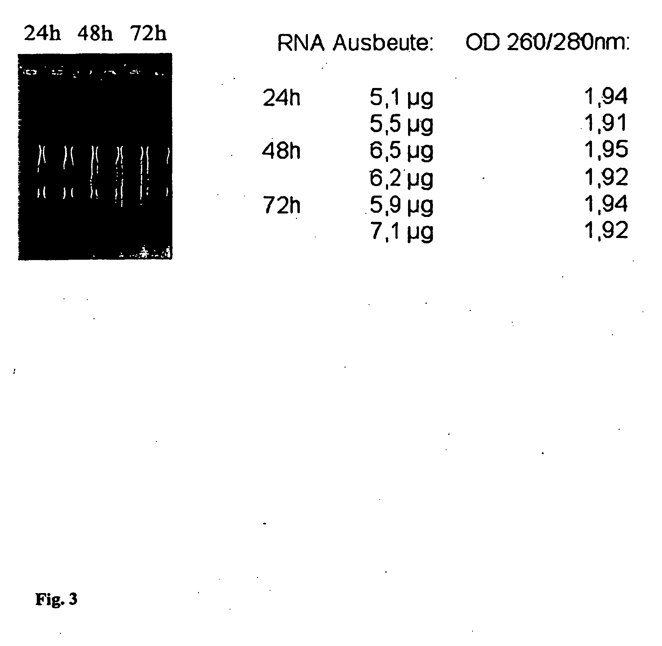

Die RNA wird in Blut für mindestens 72 Stunden ohne Degradation oder einen Ausbeuteverlust in einer Lösung aus Tetradecyltrimethylammonium-Oxalat, gepuffert mit einem Carbonsäurepuffer - z. B. 250 mM Weinsäure pH 3,0 - stabilisiert (s. Fig. 3).

2 ml Blut werden mit 2 ml eines Puffers, bestehend aus 10 % (w/v) Tetradecyltrimethylammonium-Oxalat und 250 mM Weinsäure pH 3,0 gemischt und für 24 -72 Stunden bei RT gelagert. Die Isolierung der RNA erfolgt wie in Beispiel 2 beschrieben, mit dem Unterschied, dass die Probe vor der Zentrifugation der Komplexe - bestehend aus der kationischen Verbindung und der Nukleinsäure - mit einem handelsüblichen Erythrocyten-Lysepuffer - wie z.B. dem Puffer EL der Fa. Qiagen GmbH - versetzt und danach 10 Minuten auf Eis inkubiert wird. Die RNA wird mit 80 µl RNase-freiem Wasser eluiert. Jeweils 30 µl des Eluates werden auf einem 1,2%-igen Agarose/Formaldehyd Gel aufgetrennt, bzw. in einem Spektralphotometer vermessen. Die Menge an isolierter Gesamt-RNA wird nach Verdünnung mit Wasser durch photometrische Messung der Lichtabsorption bei einer Wellenlänge von 260 nm ermittelt. Die Reinheit der so gewonnenen RNA wir durch die photometrische Bestimmung des Verhältnisses der Lichtabsorption bei 260 nm zu derjenigen bei 280 nm ermittelt.

### Beispiel 4:

### Stabilisierung der RNA in Vollblut mittels Tetradecyltrimethylammonium-Oxalat unterschiedlicher Konzentrationen, gepuffert mit Weinsäure bei pH 4,0

### Northern-Blot-Analyse

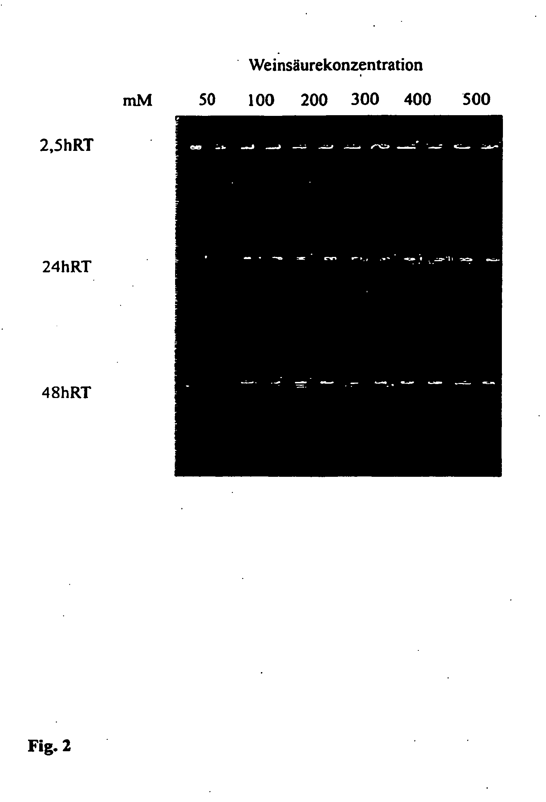

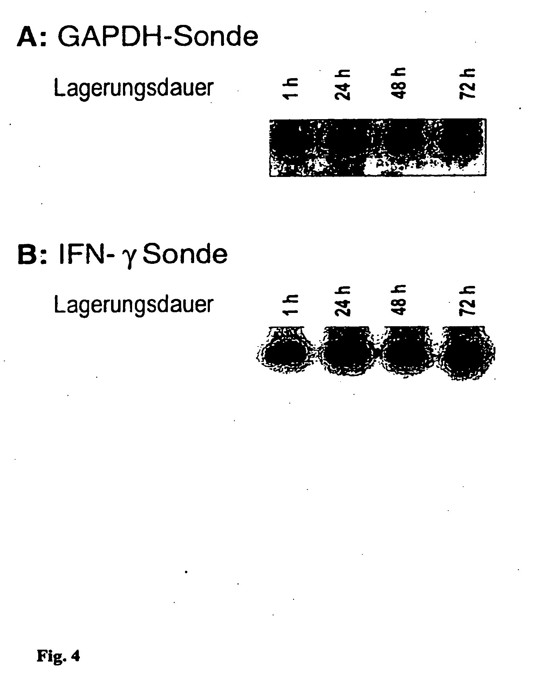

2,5 ml Blut werden mit 6,9 ml eines Puffers, bestehend aus 4 % Tetradecyltrimethylammonium-Oxalat und 200 mM Weinsäure pH 3,7 gemischt und für 1 h, 24 h, 48 h und 72 h bei RT gelagert. Zur Isolierung der RNA werden die Komplexe aus kationischer Verbindung und Nukleinsäure abzentrifugiert. Das Pellet wird einmal mit Wasser gewaschen und dann in 300 µl Lysepuffer - beispielsweise Puffer RLT der Firma QIAGEN - aufgenommen. Die weitere Probenvorbereitung erfolgt wie in Fig. 2 beschrieben. Jeweils 2,5 µg total RNA werden anschließend auf einem 1,2 %-igen denaturierenden Agarose/Formaldehyd Gel aufgetrennt. Anschließend wird die RNA auf eine Nylonmembran übertragen und über einen Zeitraum von ca. 12 h, in einem Natriumphosphat/SDS Puffer, bei 68°C, mit einer radioaktiv markierten anti-sense RNA Sonde für das GAPDH-Gen (Fig. 4A), bzw. das IFN-γ- Gen (Fig. 4B), hybridisiert. Die Membran wird mit Waschpuffern abnehmender Salzkonzentration von 2 x SSC/0,1 % SDS bis 0,1 x SSC/0,1 % SDS bei einer Temperatur von 68°C gewaschen. Die Nylonmembran wird anschließend auf einem Röntgenfilm exponiert. Sowohl das GAPDH- als auch das IFN-γ-mRNA-Signal bleibt über einen Lagerungszeitraum von über 72 h konstant. Dieses Ergebnis belegt, dass ein Abbau der m-RNA über den genannten Zeitraum nicht stattgefunden hat.

### Beispiel 5:

### Stabilisierung der genomischen DNA in Blut mittels Tetradecyltrimethylammonium-Oxalat gepuffert mit Weinsäure bei pH 3,7

Neben der zellulären RNA kann mit dem hier entwickelten Verfahren auch die genomische DNA aus Vollblut stabilisiert und anschließend durch Bindung an eine Silica-Membran isoliert werden. Fig. 5 zeigt, dass auch nach Lagerung für 72 h bei RT hochmolekulare DNA (Länge > 20 kB) isoliert wird.

2,5 ml Blut werden mit 6,9 ml einer Lösung bestehend aus 4 % (w/v) Tetradecyltrimethylammonium-Oxalat und 200 mM Weinsäure bei pH 3,7 gemischt und für 24 bzw. 72 Stunden bei RT gelagert. Zur Isolierung der DNA werden die Komplexe aus kationischer Verbindung und DNA abzentrifugiert. Das Pellet wird in 300 µl eines Natriumchlorid- und EDTA-haltigen Puffers aufgenommen, dann werden 360 µl eines kommerziell erhältlichen Guanidinium Hydrochlorid Puffers - wie z. B. der Puffer AL der Firma QIAGEN - sowie 20 µl Proteinase K zugegeben. Die Proben werden für 10 min bei 65°C inkubiert, dann werden 420 µl Ethanol zugegeben und die Probe auf eine Silica-Membran enthaltende Spin-Säule aufgetragen. Die Probe wird mittels Zentrifugation durch die Membran geführt. Die Silica-Membran wird je einmal mit einem handelsüblichen Ethanol-haltigen Guanidinium Hydrochlorid Puffer - wie z. B. der Puffer AW1 der Firma QIAGEN - und einmal mit einem Ethanol-haltigen Waschpuffer - wie z. B. der Puffer AW 2 der Firma QIAGEN - gewaschen. Die DNA wird mit 300 µl eines Tris-Puffers (pH 8) eluiert. Je 5 µl des Eluates werden auf einem 0,8 %-igen Agarose/TBE Gel aufgetrennt.

### Beispiel 6:

Verwendung der genomischen DNA in enzymatischen Reaktionen.

Fig. 6 zeigt, dass die entsprechend Beispiel 5 isolierte DNA für verschiedene enzymatische Reaktionen (Restriktion und PCR-Amplifikation) einsetzbar ist.

Die nach Lagerung für 24 bzw. 72 Stunden isolierte DNA (siehe Beispiel 5) wird in verschiedenen enzymatischen Reaktionen eingesetzt. Dies ist ein Beweis für die hohe Reinheit gute Qualität der isolierten DNA.
A) Je 2 µg der DNA werden mit 6 U der Restriktionsenzyme EcoRl (E) bzw. Hind III (H) für 3 h bei 37°C geschnitten und anschließend auf einem 0,8 %-igen Agarose/TBE-Gel aufgetrennt. Zur Kontrolle ist jeweils die ungeschnittene DNA ausgetragen.
B) Jeweils 150 bzw. 300 ng der genomischen DNA werden in eine PCR Reaktion eingesetzt (Gesamtvolumen 50 µl), bei der ein 1,1 kB langes Fragment des hugl-Gens amplifiziert wird. Die PCR-Produkte werden auf einem 1,2 %-igen Agarose/TBE-Gel aufgetrennt.

### Beispiel 7:

### RNA-Stabilisierung in Plasma mittels Tetradecyltrimethylammonium-Oxalat gemischt mit verschiedenen Additiven

Diese Experimente demonstrieren, dass der Zusatz von Carbonsäuren und anderen Additiven zu Tetradecyltrimethylammonium-Oxalat die Stabilisierung von freier RNA in Plasma im Vergleich zu RNA-Stabilisierung nur mit Tetradecyltrimethylammonium-Oxalat deutlich verbessert.

Zur Herstellung der in diesem Experiment verwendeten Lösungen wird eine Stammlösung von 30% Tetradecyltrimethylammonium-Oxalat mit jeweils einer Stammlösung von 0,5 M von Weinsäure, Zitronensäure, Tartronsäure, Bernsteinsäure, Ammoniumsulfat oder Phosphorsäure zu einer Endkonzentration von 2% oder 4% Tetradecyltrimethylammonium-Oxalat und 200 mM des Additivs vermischt. Die Stammlösungen der Additive werden vor der Mischung mit Tetradecyltrimethylammonium-Oxalat mittels Natronlauge auf den angegebenen pH-Wert eingestellt. Als Kontrolle wird eine 5% Tetradecyltrimethylammonium-Oxalat-Lösung ohne Additiv-Zusatz verwendet.

Je 0,5 ml einer jeden so erzeugten Lösung wird in ein 2 ml Eppendorf-Gefäß vorgelegt. 15 µg Gesamt-RNA aus Hela-Zellen, die zuvor z. B. mittels eines kommerziell erhältlichen RNA-Isolierungskits (z. B. RNA-Isolierungskit RNeasy^{®} Maxi-Kits der Firma QIAGEN) isoliert wird, wird in den Deckel des Eppendorf-Gefäßes pipettiert. 0,5 ml menschliches Blutplasma wird zur Lösung gegeben, der Deckel des Gefäßes geschlossen und das Gefäß zur Mischung der Flüssigkeiten fünf Mal rasch invertiert. Die Proben werden 1 Tag bei RT (ca. 20 bis 25°C) gelagert. Alle Experimente werden in Form von Doppelbestimmungen durchgeführt.

Zur Isolierung der RNA werden die Proben über einen Zeitraum von 3 min mit 25000xg zentrifugiert. Der Überstand wird abgenommen und 0,5 ml eines auf 60°C temperierten Puffers, der Guanidinium-Hydrochlorid und Nonidet P40 pH 7,0 enthält, sowie Proteinase K werden auf das Pellet gegeben. Das Pellet wird durch Vortexen gelöst und 15 min lang bei 50°C inkubiert. Anschließend wird 0,5 ml einer Ethanol-Nonidet P40-Lösung zugegeben und die Probe durch Vortexen über einen Zeitraum von ca. 5 s gemischt. Die Probe wird anschließend in eine handelsübliche Silicamembran enthaltene Spin-Säule, - wie z. B. QIAamp-Säulen der Firma QIAGEN - aufgetragen und durch Zentrifugation (1 min bei 10000 xg) durch die Membran hindurchgeführt. Die RNA bleibt an der Membran gebunden und wird anschließend zweimal mit einem alkoholhaltigen Waschpuffer, z. B. Puffer AW2 der Firma QIAGEN, gewaschen. Dabei werden die Waschpuffer jeweils durch Zentrifugation (1 min bei 10000 xg) durch die Membran geführt. Im Anschluß an die Waschung mit dem alkoholhaltigen Waschpuffer wird die Membran ohne Pufferzugabe durch eine Zentrifugation (3 min max. rpm, hier 25000xg) getrocknet. Zur Elution werden 30 µl RNase-freies Wasser auf die Membran pipettiert, um die gereinigte RNA von der Membran abzulösen. Das Eluat wird durch Zentrifugation (1 min bei 10000 xg) durch die Membran geführt und der Elutionsschritt wird zum Zwecke einer vollständigen Elution noch einmal wiederholt.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 30 µl des Eluates eingesetzt. Das Ergebnis ist in Fig. 7 wiedergegeben. Die Beladung der Gelspuren ist in Tabelle 2 zusammengefaßt.

**Tabelle 2: Zusammenfassung der in Fig. 7 dargestellten Proben.**

| Proben Nr. | Endkonzentration an Tetradecyltrimethylammonium-Oxalat | Additiv |
|---|---|---|
| 1,2 | 4% | Zitronensäure pH 4 |
| 3,4 | 4% | Zitronensäure pH 5 |
| 5,6 | 4% | Zitronensäure pH 6 |
| 7,8 | 4% | Weinsäure pH 3 |
| 9,10 | 4% | Weinsäure pH 4 |
| 11,12 | 4% | Bernsteinsäure pH 4 |
| 13,14 | 4% | Tartronsäure pH 3 |
| 15,16 | 4% | Tartronsäure pH 4 |
| 17,18 | 4% | Phosphorsäure pH 3 |
| 19,20 | 4% | Phosphorsäure pH 4 |
| 21,22 | 4% | Phosphorsäure pH 5 |
| 23,24 | 2% | Zitronensäure pH 3 |
| 25,26 | 2% | Zitronensäure pH 4 |
| 27,28 | 2% | Weinsäure pH 3 |
| 29,30 | 2% | Weinsäure pH 4 |
| 31,32 | 2% | Bernsteinsäure pH 4 |
| 33,34 | 2% | Phosphorsäure pH 2 |
| 35,36 | 2% | Phosphorsäure pH 3 |
| 37,38 | 2% | Phosphorsäure pH 4 |
| 39,40 | 2% | Phosphorsäure pH 5 |
| 41,42 | 4% | Ammoniumsulfat pH 2 |
| 43,44 | 5% | - |

Spur 45 enthält zum Vergleich der RNA-Qualität der einzelnen Proben 3,75 µg der für diese Versuche eingesetzten Gesamt-RNA aus Hela-Zellen.

Die gelelektrophoretische Auftrennung der für dieses Experiment eingesetzten Hela-Gesamt-RNA zeigt nach Anfärbung mit Ethidiumbromid die intakte 28S und 18S rRNA. Die obere der sichtbaren rRNA-Banden (28S rRNA) ist dabei deutlich intensiver und dicker als die untere rRNA-Bande (18S rRNA), was ein typisches Merkmal intakter, nicht abgebauter RNA darstellt. Der Vergleich der eintägig in mit 5% Tetradecyltrimethylammonium-Oxalat ohne Additiv-Zusatz vermischtem Plasma gelagerten Hela-Gesamt-RNA mit der RNA, die nach eintägiger Lagerung aus in mit Tetradecyltrimethylammonium-Oxalat und verschiedenen Additiven vermischtem Plasma isoliert wird, zeigt deutlich, dass der Zusatz von Additiven die RNA-Stabilisierung verbessert. Wird RNA ohne Zusatz einer stabilisierenden Verbindung zu Plasma gegeben, führt dies bekannterweise zu einem vollständigen RNA-Abbau binnen weniger Minuten.

### Beispiel 8:

### RNA-Stabilisierung in Plasma mittels Tetradecyltrimethylammonium-Oxalat gemischt mit Wein- bzw. Tartronsäure über verschiedene Zeiträume

Diese Experimente zeigen, dass die RNA durch Tetradecyltrimethylammonium-Oxalat-Additiv-Mischungen in Plasma bis zu mindestens 14 Tagen stabilisiert wird.

Zur Herstellung der in diesem Experiment verwendeten Lösungen wird eine Stammlösung von 30% Tetradecyltrimethylammonium-Oxalat mit jeweils einer Stammlösung von 0,5 M von Weinsäure pH 3 oder Tartronsäure pH 3 zu einer Endkonzentration von 6% oder 8% Tetradecyltrimethylammonium-Oxalat und 200 mM des Additivs vermischt.

Je 0,5 ml einer jeden so erzeugten Lösung wird in ein 2 ml Eppendorf-Gefäß vorgelegt. 15 µg Gesamt-RNA aus Hela-Zellen, die zuvor mittels eines kommerziell erhältlichen RNA-lsolierungskits - wie z. B. RNeasy^{®} Maxi-Kits der Firma QIAGEN - isoliert wird, wird in den Deckel des Eppendorf-Gefäßes pipettiert. 0,5 ml menschliches Blut-Plasma wird zur Lösung gegeben, der Deckel des Gefäßes geschlossen und das Gefäß zur Mischung der Flüssigkeiten fünf Mal rasch invertiert. Die Proben werden 3, 7, 10 und 14 Tage bei RT (ca. 20 bis 25°C) gelagert. Alle Experimente werden in Form von Doppelbestimmungen durchgeführt.

Die RNA-Isolierung erfolgt wie in Beispiel 7 beschrieben.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 30 µl des Eluates eingesetzt. Das Ergebnis ist in Fig. 8 wiedergegeben. Die Beladung der Gelspuren ist in Tabelle 3 zusammengefaßt.

**Tabelle 3: Zusammenfassung der in Fig. 8 dargestellten Proben.**

| Proben Nr. | Konzentration Tetradecyltrimethylammoniumoxalatim Puffer | Additiv | Lagerungsdauer |
|---|---|---|---|
| 1,2 | 6% | Weinsäure pH 3 | 3 Tage |
| 3,4 | 8% | Weinsäure pH 3 | 3 Tage |
| 5,6 | 6% | Tartronsäure pH 3 | 3 Tage |
| 7,8 | 8% | Tartronsäure pH 3 | 3 Tage |
| 9,10 | 6% | Weinsäure pH 3 | 7 Tage |
| 11,12 | 8% | Weinsäure pH 3 | 7 Tage |
| 13,14 | 6% | Tartronsäure pH 3 | 7 Tage |
| 15,16 | 8% | Tartronsäure pH 3 | 7 Tage |
| 17,18 | 6% | Weinsäure pH 3 | 10 Tage |
| 19,20 | 8% | Weinsäure pH 3 | 10 Tage |
| 21,22 | 6% | Tartronsäure pH 3 | 10 Tage |
| 23,24 | 8% | Tartronsäure pH 3 | 10 Tage |
| 25,26 | 6% | Weinsäure pH 3 | 14 Tage |
| 27,28 | 8% | Weinsäure pH 3 | 14 Tage |
| 29,30 | 6% | Tartronsäure pH 3 | 14 Tage |
| 31,32 | 8% | Tartronsäure pH 3 | 14 Tage |

Spur "K" enthält zum Vergleich der RNA-Qualität der einzelnen Proben 3,75 µg der für diese Versuche eingesetzten Gesamt-RNA aus Hela-Zellen.

Die gelelektrophoretische Auftrennung der zeigt nach Anfärbung mit Ethidiumbromid die intakte 28S und 18S rRNA-Banden, auch nach bis zu 14-tägiger Lagerung der Hela-Gesamt-RNA in Plasma, das mit Tetradecyltrimethylammonium-Oxalat und Weinsäure bzw. Tartronsäure pH 3 vermischt wurde.

### Beispiel 9:

### RNA-Stabilisierung in 1 ml Plasma mittels Tetradecyltrimethylammonium-Oxalat gemischt mit verschiedenen Additiven

Diese Experimente zeigen, dass die RNA durch Tetradecyltrimethylammonium-Oxalat-Additiv-Mischungen auch in einem größeren Plasmavolumen möglich ist.

Zur Herstellung der in diesem Experiment verwendeten Lösungen wird eine Stammlösung von 30% Tetradecyltrimethylammonium-Oxalat mit jeweils einer Stammlösung von 0,5 M von Weinsäure, bei pH 3 oder pH 4, Tartronsäure bei pH 3 oder pH 4 oder von Phosphorsäure bei pH 3 oder pH 4 zu einer Endkonzentration von 4 % Tetradecyltrimethylammonium-Oxalat und 200 mM des Additivs vermischt.

Je 1 ml einer jeden so erzeugten Lösung wird in ein 2 ml Eppendorf-Gefäß vorgelegt. 15 µg Gesamt-RNA aus Hela-Zellen, die zuvor z. B. mittels des RNA-Isolierungskits RNeasy^{®} Maxi-Kits der Firma QIAGEN isoliert wird, wird in den Deckel des Eppendorf-Gefäßes pipettiert. 1 ml menschliches Blut-Plasma wird zur Lösung gegeben, der Deckel des Gefäßes geschlossen und das Gefäß zur Mischung der Flüssigkeiten fünf Mal rasch invertiert. Die Proben werden 3 Tage bei RT (ca. 20 bis 25°C) gelagert. Alle Experimente werden in Form von Doppelbestimmungen durchgeführt.

Die RNA-lsolierung erfolgt wie in Beispiel 7 beschrieben.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 30 µl des Eluates eingesetzt. Das Ergebnis ist in Fig. 9 wiedergegeben. Die Beladung der Gelspuren ist in Tabelle 4 zusammengefaßt.

**Tabelle 4: Zusammenfassung der in Fig. 9 dargestellten Proben**

| Proben Nr. | Additiv |
|---|---|
| 1,2 | Weinsäure pH 3 |
| 3,4 | Weinsäure pH 4 |
| 5,6 | Phosphorsäure pH 3 |
| 7,8 | Phosphorsäure pH 4 |
| 9,10 | Tartronsäure pH 3 |
| 11,12 | Tartronsäure pH 4 |

Spur 13 enthält zum Vergleich der RNA-Qualität der einzelnen Proben 3,75 µg der für diese Versuche eingesetzten Gesamt-RNA aus Hela-Zellen.

Die gelelektrophoretische Auftrennung der zeigt nach Anfärbung mit Ethidiumbromid die intakte 28S und 18S rRNA-Banden. Die RNA wird somit auch in einem größeren Plasmavolumen durch die Tetradecyltrimethylammonium-Oxalat-Additiv-Mischung stabilisiert.

### Beispiel 10:

### RNA-Stabilisierung in Hela-Zellen mittels Tetradecyltrimethylammonium-Oxalat gemischt mit verschiedenen Additiven

Diese Experimente demonstrieren, dass Mischungen aus Tetradecyltrimethylammonium-Oxalat mit verschiedenen Additiven die Stabilisierung von RNA in Hela-Zellen über eine Lagerungsdauer von bis zu 14 Tagen bei RT - ermöglichen.

Zur Herstellung der in diesem Experiment verwendeten Lösungen wird eine Stammlösung von 20 % oder 30% Tetradecyltrimethylammonium-Oxalat mit jeweils einer Stammlösung von 0,5 M von Weinsäure, Zitronensäure, Tartronsäure, Ammoniumsulfat oder Phosphorsäure zu einer Endkonzentration von 2 % oder 4 % Tetradecyltrimethylammonium-Oxalat und 200 mM des Additivs vermischt. Die Stammlösungen der Additive werden vor der Mischung mit Tetradecyltrimethylammonium-Oxalat mittels Natronlauge bzw. Schwefelsäure auf den angegebenen pH-Wert eingestellt.

Je 1x10⁶ Hela-Zellen, die direkt zuvor aus der Zellkultur geerntet und mit PBS gewaschen werden, werden durch Zentrifugation (1 min bei 120 xg) pelletiert und der Überstand entfernt. Zu den Zellen werden jeweils 300 µl der in Tabelle 4 genannten Lösungen gegeben und die Proben durch Vortexen gemischt und dabei die Zellen resuspendiert. Die Proben werden 3, 7, 10 und 14 Tage bei RT (ca. 20 bis 25°C) gelagert. Alle Experimente werden in Form von Doppelbestimmungen durchgeführt.

Zur RNA-Isolierung werden die Zellen durch dreiminütige Zentrifugation bei 1200 xg pelletiert und der Überstand abgenommen. Das Pellet wird in 600 µl eines handelsüblichen Guanidinium-Isothiocyanat-Puffers - wie z. B. RLT-Puffer der Firma QIAGEN - durch mehrmaliges Auf- und Abpipettieren oder durch Vortexen über einen Zeitraum von ca. 10 s oder länger re-suspendiert. Anschließend wird 1 Volumen (600 µl) 70 %-iges Ethanol zugefügt und durch mehrmaliges Auf- und Abpipettieren oder durch Vortexen über einen Zeitraum von ca. 5 s gemischt. Das Lysat wird anschließend in eine handelsübliche Silicamembran enthaltene Spin-Säule - wie z. B. RNeasy-Säulen der Firma QIAGEN - aufgetragen und durch Zentrifugation (1 min bei 10000 xg) durch die Membran hindurchgeführt. Die RNA bleibt an der Membran gebunden und wird anschließend wird mit einem ersten handelsüblichen Guanidinium-Isothiocyanat-haltigen Waschpuffer - beispielsweise mit dem Puffer RW1 der Firma QIAGEN - und danach mit einem zweiten alkoholhaltigen Waschpuffer, z. B. Puffer RPE der Firma QIAGEN, gewaschen. Dabei werden die Waschpuffer jeweils durch Zentrifugation (1 min bei 10000 xg) durch die Membran geführt. Die Waschung mit dem zweiten alkoholhaltigen Waschpuffer wird mit einem geringeren Volumen wiederholt wobei gleichzeitig die Membran durch die Zentrifugation (2 min max. rpm, hier 20000xg) getrocknet wird. Zur Elution werden 40 µl RNase-freies Wasser auf die Membran pipettiert, um die gereinigte RNA von der Membran abzulösen. Durch Zentrifugation (1 min bei 10000 xg) wird das Eluat durch die Membran hindurchgeführt und der Elutionsschritt wird zum Zwecke einer vollständigen Elution noch einmal wiederholt.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 20 µl des Eluates eingesetzt. Das Ergebnis ist in Fig. 10 wiedergegeben. Die Proben sind in Tabelle 5 zusammengefaßt, wobei alle Proben jeweils 2x durchgeführt und dargestellt worden sind mit Ausnahme der Proben 14, 40, 66 und 92 die 1x durchgeführt und dargestellt worden sind.

**Tabelle 5: Zusammenfassung der in Fig. 10 dargestellten Proben**

| Proben Nr. | Endkonzentration an Tetradecyltrimethylammonium-Oxalat | Additiv | finaler pH-Wert der Mischung aus Tetradecyltrimethylammonium-Oxalat und Additv ca. | Lagerdauer |
|---|---|---|---|---|
| 1 | 4% | Weinsäure pH 3 | 3,4 | 3 Tage |
| 2 | 4% | Weinsäure pH 4 | 4,3 | 3 Tage |
| 3 | 4% | Weinsäure pH 5 | 5,3 | 3 Tage |
| 4 | 4% | Weinsäure pH 6 | 6,0 | 3 Tage |
| 5 | 4% | Weinsäure pH 7 | 7,3 | 3 Tage |
| 6 | 4% | Phosphorsäure pH 3 | 4,3 | 3 Tage |
| 7 | 4% | Phosphorsäure pH 4 | 4,9 | 3 Tage |
| 8 | 4% | Phosphorsäure pH 5 | 6,0 | 3 Tage |
| 9 | 4% | Phosphorsäure pH 6 | 6,3 | 3 Tage |
| 10 | 4% | Phosphorsäure pH 7 | 7,1 | 3 Tage |
| 11 | 4% | Ammoniumsulfat pH 2 | 4,1 | 3 Tage |
| 12 | 4% | Ammoniumsulfat pH 3 | 5,2 | 3 Tage |
| 13 | 4% | Ammoniumsulfat pH 4 | 6,0 | 3 Tage |
| 14 | 4% | Ammoniumsulfat pH 5 | 6,1 | 3 Tage |
| 15 | 4% | Zitronensäure pH 3 | 3,3 | 3 Tage |
| 16 | 4% | Zitronensäure pH 4 | 4,3 | 3 Tage |
| 17 | 4% | Zitronensäure pH 5 | 5,4 | 3 Tage |
| 18 | 4% | Zitronensäure pH 6 | 6,3 | 3 Tage |
| 19 | 4% | Zitronensäure pH 7 | 7,5 | 3 Tage |
| 20 | 4% | Tartronsäure pH 3 | 3,6 | 3 Tage |
| 21 | 4% | Tartronsäure pH 4 | 4,4 | 3 Tage |
| 22 | 4% | Tartronsäure pH 5 | 5,3 | 3 Tage |
| 23 | 4% | Tartronsäure pH 6 | 5,9 | 3 Tage |
| 24 | 4% | Tartronsäure pH 7 | 7,3 | 3 Tage |
| 25 | 2% | Weinsäure pH 3 | nb | 3 Tage |
| 26 | 2% | Weinsäure pH 6 | nb | 3 Tage |
| 27 | 4% | Weinsäure pH 3 | 3,4 | 7 Tage |
| 28 | 4% | Weinsäure pH 4 | 4,3 | 7 Tage |
| 29 | 4% | Weinsäure pH 5 | 5,3 | 7 Tage |
| 30 | 4% | Weinsäure pH 6 | 6,0 | 7 Tage |
| 31 | 4% | Weinsäure pH 7 | 7,3 | 7 Tage |
| 32 | 4% | Phosphorsäure pH 3 | 4,3 | 7 Tage |
| 33 | 4% | Phosphorsäure pH 4 | 4,9 | 7 Tage |
| 34 | 4% | Phosphorsäure pH 5 | 6,0 | 7 Tage |
| 35 | 4% | Phosphorsäure pH 6 | 6,3 | 7 Tage |
| 36 | 4% | Phosphorsäure pH 7 | 7,1 | 7 Tage |
| 37 | 4% | Ammoniumsulfat pH 2 | 4,1 | 7 Tage |
| 38 | 4% | Ammoniumsulfat pH 3 | 5,2 | 7 Tage |
| 39 | 4% | Ammoniumsulfat pH 4 | 6,0 | 7 Tage |
| 40 | 4% | Ammoniumsulfat pH 5 | 6,1 | 7 Tage |
| 41 | 4% | Zitronensäure pH 3 | 3,3 | 7 Tage |
| 42 | 4% | Zitronensäure pH 4 | 4,3 | 7 Tage |
| 43 | 4% | Zitronensäure pH 5 | 5,4 | 7 Tage |
| 44 | 4% | Zitronensäure pH 6 | 6,3 | 7 Tage |
| 45 | 4% | Zitronensäure pH 7 | 7,5 | 7 Tage |
| 46 | 4% | Tartronsäure pH 3 | 3,6 | 7 Tage |
| 47 | 4% | Tartronsäure pH 4 | 4,4 | 7 Tage |
| 48 | 4% | Tartronsäure pH 5 | 5,3 | 7 Tage |
| 49 | 4% | Tartronsäure pH 6 | 5,9 | 7 Tage |
| 50 | 4% | Tartronsäure pH 7 | 7,3 | 7 Tage |
| 51 | 2% | Weinsäure pH 3 | nb | 7 Tage |
| 52 | 2% | Weinsäure pH 6 | nb | 7 Tage |
| 53 | 4% | Weinsäure pH 3 | 3,4 | 10 Tage |
| 54 | 4% | Weinsäure pH 4 | 4,3 | 10 Tage |
| 55 | 4% | Weinsäure pH 5 | 5,3 | 10 Tage |
| 56 | 4% | Weinsäure pH 6 | 6,0 | 10 Tage |
| 57 | 4% | Weinsäure pH 7 | 7,3 | 10 Tage |
| 58 | 4% | Phosphorsäure pH 3 | 4,3 | 10 Tage |
| 59 | 4% | Phosphorsäure pH 4 | 4,9 | 10 Tage |
| 60 | 4% | Phosphorsäure pH 5 | 6,0 | 10 Tage |
| 61 | 4% | Phosphorsäure pH 6 | 6,3 | 10 Tage |
| 62 | 4% | Phosphorsäure pH 7 | 7,1 | 10 Tage |
| 63 | 4% | Ammoniumsulfat pH 2 | 4,1 | 10 Tage |
| 64 | 4% | Ammoniumsulfat pH 3 | 5,2 | 10 Tage |
| 65 | 4% | Ammoniumsulfat pH 4 | 6,0 | 10 Tage |
| 66 | 4% | Ammoniumsulfat pH 5 | 6,1 | 10 Tage |
| 67 | 4% | Zitronensäure pH 3 | 3,3 | 10 Tage |
| 68 | 4% | Zitronensäure pH 4 | 4,3 | 10 Tage |
| 69 | 4% | Zitronensäure pH 5 | 5,4 | 10 Tage |
| 70 | 4% | Zitronensäure pH 6 | 6,3 | 10 Tage |
| 71 | 4% | Zitronensäure pH 7 | 7,5 | 10 Tage |
| 72 | 4% | Tartronsäure pH 3 | 3,6 | 10 Tage |
| 73 | 4% | Tartronsäure pH 4 | 4,4 | 10 Tage |
| 74 | 4% | Tartronsäure pH 5 | 5,3 | 10 Tage |
| 75 | 4% | Tartronsäure pH 6 | 5,9 | 10 Tage |
| 76 | 4% | Tartronsäure pH 7 | 7,3 | 10 Tage |
| 77 | 2% | Weinsäure pH 3 | nb | 10 Tage |
| 78 | 2% | Weinsäure pH 6 | nb | 10 Tage |
| 79 | 4% | Weinsäure pH 3 | 3,4 | 14 Tage |
| 80 | 4% | Weinsäure pH 4 | 4,3 | 14 Tage |
| 81 | 4% | Weinsäure pH 5 | 5,3 | 14 Tage |
| 82 | 4% | Weinsäure pH 6 | 6,0 | 14 Tage |
| 83 | 4% | Weinsäure pH 7 | 7,3 | 14 Tage |
| 84 | 4% | Phosphorsäure pH 3 | 4,3 | 14 Tage |
| 85 | 4% | Phosphorsäure pH 4 | 4,9 | 14 Tage |
| 86 | 4% | Phosphorsäure pH 5 | 6,0 | 14 Tage |
| 87 | 4% | Phosphorsäure pH 6 | 6,3 | 14 Tage |
| 88 | 4% | Phosphorsäure pH 7 | 7,1 | 14 Tage |
| 89 | 4% | Ammoniumsulfat pH 2 | 4,1 | 14 Tage |
| 90 | 4% | Ammoniumsulfat pH 3 | 5,2 | 14 Tage |
| 91 | 4% | Ammoniumsulfat pH 4 | 6,0 | 14 Tage |
| 92 | 4% | Ammoniumsulfat pH 5 | 6,1 | 14 Tage |
| 93 | 4% | Zitronensäure pH 3 | 3,3 | 14 Tage |
| 94 | 4% | Zitronensäure pH 4 | 4,3 | 14 Tage |
| 95 | 4% | Zitronensäure pH 5 | 5,4 | 14 Tage |
| 96 | 4% | Zitronensäure pH 6 | 6,3 | 14 Tage |
| 97 | 4% | Zitronensäure pH | 7 7,5 | 14 Tage |
| 98 | 4% | Tartronsäure pH 3 | 3,6 | 14 Tage |
| 99 | 4% | Tartronsäure pH 4 | 4,4 | 14 Tage |
| 100 | 4% | Tartronsäure pH 5 | 5,3 | 14 Tage |
| 1.01 | 4% | Tartronsäure pH 6 | 5,9 | 14 Tage |
| 102 | 4% | Tartronsäure pH 7 | 7,3 | 14 Tage |
| 103 | 2% | Weinsäure pH 3 | nb | 14 Tage |
| 104 | 2% | Weinsäure pH 6 | nb | 14 Tage |

Die Proben "K" zeigen eine Gesamt-RNA, die ohne vorherige Lagerung mit Hilfe eines Isolierungskits - wie z. B. dem RNeasy^{®} Mini Kits der Firma QIAGEN - aus je 1 x 10⁶ Hela-Zellen isoliert wird (= Positiv-Kontrolle). Die Proben "a", "b", "c" und "d" zeigen eine Gesamt-RNA, die nach 3, 7, 10 bzw. 14 Tagen Lagerung von je 1 x 10⁶ Hela-Zellen in PBS - ohne Zusätze - wie oben beschrieben isoliert wird.

Die Menge an isolierter Gesamt-RNA wird nach Verdünnung in Wasser durch photometrische Messung der Lichtabsorption bei einer Wellenlänge von 260 nm ermittelt. Die Reinheit der so gewonnenen RNA wird durch die photometrische Bestimmung des Verhältnisses der Lichtabsorption bei 260 nm zu derjenigen bei 280 nm bestimmt. Die Ergebnisse der Isolierungen sind in der nachfolgenden Tabelle 6 dargestellt. Es werden jeweils die Mittelwerte der Doppelbestimmung angegeben.

**Tabelle 6: RNA-Ausbeute der nach Beispiel 10 aus in Tetradecyltrimethylammonium-Oxalat gemischt mit verschiedenen Additiven gelagerten Hela-Zellen isolierten Gesamt-RNA**

| Additiv | Lagerungsdauer (Tage) | RNA-Ausbeute (µg) | E₂₆₀/ E₂₈₀ |
|---|---|---|---|
| Weinsäure pH 5 | 3 | 28,7 | 1,84 |
| Weinsäure pH 5 | 7 | 30,7 | 1,86 |
| Weinsäure pH 5 | 10 | 33,4 | 1,90 |
| Weinsäure pH 5 | 14 | 56,4 | 1,94 |
| Weinsäure pH 6 | 3 | 38,4 | 1,92 |
| Weinsäure pH 6 | 7 | 55,5 | 2,0 |
| Weinsäure pH 6 | 10 | 36,1 | 1,93 |
| Weinsäure pH 6 | 14 | 36,9 | 1,94 |
| Phosphorsäure pH 5 | 3 | 39,5 | 1,89 |
| Phosphorsäure pH 5 | 7 | 27,1 | 1,91 |
| Phosphorsäure pH 5 | 10 | 36,9 | 1,89 |
| Phosphorsäure pH 5 | 14 | 40,2 | 1,85 |
| Phosphorsäure pH 6 | 3 | 25,6 | 1,98 |
| Phosphorsäure pH 6 | 7 | 29,2 | 1,89 |
| Phosphorsäure pH 6 | 10 | 34,2 | 1,88 |
| Phosphorsäure pH 6 | 14 | 40,9 | 1,95 |
| Tartronsäure pH 5 | 3 | 24,7 | 1,95 |
| Tartronsäure pH 5 | 7 | 30,8 | 1,91 |
| Tartronsäure pH 5 | 10 | 30,4 | 1,90 |
| Tartronsäure pH 5 | 14 | 30,8 | 1,95 |
| Tartronsäure pH 6 | 3 | 30,6 | 1,96 |
| Tartronsäure pH 6 | 7 | 31,0 | 1,90 |
| Tartronsäure pH 6 | 10 | 34,0 | 1,95 |
| Tartronsäure pH 6 | 14 | 32,0 | 1,92 |
| Ammoniumsulfat pH 5 | 3 | 31,5 | 1,98 |
| Ammoniumsulfat pH 5 | 7 | 27,1 | 1,88 |
| Ammoniumsulfat pH 5 | 10 | 35,7 | 1,93 |
| Ammoniumsulfat pH 5 | 14 | 35,5 | 1,92 |
| Zitronensäure pH 6 | 3 | 24,4 | 1,91 |
| Zitronensäure pH 6 | 7 | 31,5 | 1,94 |
| Zitronensäure pH 6 | 10 | 32,5 | 1,94 |
| Zitronensäure pH 6 | 14 | 39,2 | 1,94 |
| Positiv-Kontrolle | 0 | 33,2 | 1,90 |

Die gelelektrophoretische Auftrennung zeigt nach Anfärbung mit Ethidiumbromid die intakte 28S und 18S rRNA-Banden in den Positiv-Kontrollen. Die obere der rRNA-Banden (28S rRNA) ist dabei deutlich intensiver und dicker als die untere rRNA-Bande (18S rRNA), was ein typisches Merkmal intakter, nicht abgebauter RNA darstellt. Nach 3-tägiger Lagerung der Zellen in PBS ist die RNA z. T. abgebaut, da die beiden rRNA-Banden gleiche Intensität zeigen und deutlich weniger RNA sichtbar ist. Nach 7-tägiger oder längerer Lagerung ist keine RNA mehr sichtbar. Im Gegensatz dazu wird die RNA in Hela-Zellen durch Tetradecyltrimethylammonium-Oxalat gemischt mit verschiedenen Additiven bis zu 14 Tagen stabilisiert. Dies wird durch die OD-Messung bestimmte RNA-Ausbeute und -Reinheit bestätigt. Die Stabilisierung wird vom pH-Wert beeinflußt. Dabei sind finale pH-Werte in der Mischung, d. h. nach Mischung von Tetradecyltrimethylammonium-Oxalat und Additiv definierten pH-Wertes, von größer 4 bevorzugt.

### Beispiel 11:

### RNA-Stabilisierung in unterschiedlichen Mengen von Hela-Zellen

Diese Experimente zeigen, dass die Stabilisierung von RNA in Hela-Zellen mittels Mischungen von Tetradecyltrimethylammonium-Oxalat mit Additiven unabhängig von der Anzahl der eingesetzten Zellen erfolgt.

Zur Herstellung der in diesem Experiment verwendeten Lösung wird eine Stammlösung von 20% Tetradecyltrimethylammonium-Oxalat mit einer Stammlösung von 0,5 M Weinsäure bei pH 6 zu einer Endkonzentration von 4% Tetradecyltrimethylammonium-Oxalat und 200 mM des Additivs vermischt. Die Stammlösung des Additivs wird vor der Mischung mit Tetradecyltrimethylammonium-Oxalat mittels Natronlauge auf den angegebenen pH-Wert eingestellt.

Je 1 x10⁵, 5x10⁵, 1x10⁶ und 5x10⁶ Hela-Zellen, die direkt zuvor aus der Zellkultur geerntet und mit PBS gewaschen werden, werden durch Zentrifugation (1 min bei 120 xg) pelletiert und der Überstand entfernt. Zu den Zellen wird jeweils 300 µl Lösung mit 4% Tetradecyltrimethylammonium-Oxalat und 200 mM Weinsäure gegeben und die Proben durch Vortexen gemischt und dabei die Zellen resuspendiert. Die Proben werden 15 min bzw. 1 Tag bei RT (ca. 20 bis 25°C) gelagert. Alle Experimente werden in Form von Doppelbestimmungen durchgeführt.

Die RNA-Isolierung erfolgt wie in Beispiel 10 beschrieben.

Als Kontrollen werden je 1 x10⁵, 5x10⁵, 1x10⁶ und 5x10⁶ Hela-Zellen ohne vorherige Behandlung mit 4% Tetradecyltrimethylammonium-Oxalat, 200 mM Weinsäure und ohne Lagerung zur RNA-Isolierung wie oben beschrieben eingesetzt.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 20 µl des Eluates eingesetzt. Das Ergebnis ist in Fig. 11 wiedergegeben. Die Proben sind in Tabelle 7 zusammengefaßt, wobei alle Proben jeweils 2x durchgeführt und dargestellt worden sind.

**Tabelle 7: Zusammenfassung der in Fig. 11 dargestellten Proben**

| Proben Nr. | Zellanzahl | Lagerung |
|---|---|---|
| 1,2 | 1x10⁵ | - |
| 3,4 | 1x10⁵ | 15 min |
| 5,6 | 1x10⁵ | 1 Tag |
| 7,8 | 5x10⁵ | - |
| 9,10 | 5x10⁵ | 15 min |
| 11,12 | 5x10⁵ | 1 Tag |
| 13,14 | 1x10⁶ | - |
| 15,16 | 1x10⁶ | 15 min |
| 17,18 | 1x10⁶ | 1 Tag |
| 19,20 | 5x10⁶ | - |
| 21,22 | 5x10⁶ | 15 min |
| 23,24 | 5x10⁶ | 1 Tag |

Die Menge an isolierter Gesamt-RNA wird nach Verdünnung in Wasser durch photometrische Messung der Lichtabsorption bei einer Wellenlänge von 260 nm ermittelt. Die Reinheit der so gewonnenen RNA wird durch die photometrische Bestimmung des Verhältnisses der Lichtabsorption bei 260 nm zu derjenigen bei 280 nm bestimmt. Die Ergebnisse der Isolierungen sind in der nachfolgenden Tabelle 8 dargestellt. Es werden jeweils die Mittelwerte der Doppelbestimmung angegeben.

**Tabelle 8: RNA-Ausbeute der nach Beispiel 11 aus in 4% Tetradecyl-trimethylammonium-Oxalat, 200 mM Weinsäure gelagerten Hela-Zellen isolierten Gesamt-RNA**

| Zellzahl | Lagerung | RNA-Ausbeute (µg) | E₂₆₀/E₂₈₀ |
|---|---|---|---|
| 1x10⁵ | - | 3,0 | 2,04 |
| 1x10⁵ | 15 min | 3,1 | 1,92 |
| 1x10⁵ | 1 Tag | 3,5 | 1,97 |
| 5x10⁵ | - | 16,2 | 1,83 |
| 5x10⁵ | 15 min | 15,2 | 1,85 |
| 5x10⁵ | 1 Tag | 16,0 | 1,86 |
| 1x10⁶ | - | 28,2 | 1,75 |
| 1x10⁶ | 15 min | 28,2 | 1,73 |
| 1x10⁶ | 1 Tag | 34,4 | 1,77 |
| 5x10⁶ | - | 107,3 | 1,64 |
| 5x10⁶ | 15 min | 91,3 | 1,61 |
| 5x10⁶ | 1 Tag | 122,6 | 1,61 |

Die gelelektrophoretische Auftrennung zeigt nach Anfärbung mit Ethidiumbromid die intakte 28S und 18S rRNA-Banden in den gelagerten ebenso wie in den nicht gelagerten Kontrollproben. Dabei ist kein Unterschied zwischen den nicht-gelagerten Kontrollen und den gelagerten Proben zu erkennen. Ebenso bestätigt die durch OD-Messung bestimmte RNA-Ausbeute und -Reinheit, dass die RNA-Stabilisierung in verschiedenen Zellmengen gleichermaßen erfolgt, ohne Verringerung der RNA-Ausbeuten oder RNA-Reinheit. Die mit zunehmender Zellzahl abnehmenden E₂₆₀/E₂₈₀-Quotienten sind darauf zurückzuführen, dass diese Messungen in Wasser und nicht in einem gepufferten System durchgeführt wurden.

### Beispiel 12:

### RNA-Stabilisierung in Macrophagen

Diese Experimente demonstrieren, dass RNA in verschiedenen Zelltypen eingesetzt werden kann. Die in diesem Experiment eingesetzten Macrophagen enthalten mehr RNasen als die zuvor verwendeten Hela-Zellen, wodurch der Abbau von RNA in den Zellen forciert wird.

Zur Herstellung der in diesem Experiment verwendeten Lösungen wird eine Stammlösung von 20% Tetradecyltrimethylammonium-Oxalat mit einer Stammlösung von 0,5 M Weinsäure pH 5, 0,5 M Tartronsäure pH 5 oder 0,5 M Phosphorsäure pH 5 zu einer Endkonzentration von 4% Tetradecyltrimethylammonium-Oxalat und 200 mM des Additivs vermischt. Die Stammlösung des Additivs wird vor der Mischung mit Tetradecyltrimethylammonium-Oxalat mittels Natronlauge auf den angegebenen pH-Wert eingestellt.

Je 1x10⁶ Hela-Zellen, die direkt zuvor aus der Zellkultur geerntet und mit PBS gewaschen werden, werden durch Zentrifugation (1 min bei 120 xg) pelletiert und der Überstand entfernt. Zu den Zellen wird jeweils 300 µl Lösung mit 4% Tetradecyltrimethylammonium-Oxalat und 200 mM Additiv gegeben und die Proben durch Vortexen gemischt und dabei die Zellen resuspendiert. Die Proben werden 2 Tage, 6 Tage, 9 Tage bzw. 14 Tage bei RT (ca. 20 bis 25°C) gelagert. Alle Experimente werden in Form von Doppelbestimmungen durchgeführt.

Die RNA-Isolierung erfolgt wie in Beispiel 10 beschrieben.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 20 µl des Eluates eingesetzt. Das Ergebnis ist in Fig. 12 wiedergegeben. Die Proben sind in Tabelle 9 zusammengefaßt, wobei alle Proben jeweils 2x durchgeführt und dargestellt worden sind.

**Tabelle 9: Zusammenfassung der in Fig. 12 dargestellten Proben**

| Proben Nr. | Additiv | Lagerung |
|---|---|---|
| 1,2 | 200 mM Phosphorsäure pH 5 | 2 Tage |
| 3,4 | 200 mM Phosphorsäure pH 5 | 6 Tage |
| 5,6 | 200 mM Phosphorsäure pH 5 | 9 Tage |
| 7,8 | 200 mM Phosphorsäure pH 5 | 14 Tage |
| 9,10 | 200 mM Tartronsäure pH 5 | 2 Tage |
| 11,12 | 200 mM Tartronsäure pH 5 | 6 Tage |
| 13,14 | 200 mM Tartronsäure pH 5 | 9 Tage |
| 15,16 | 200 mM Tartronsäure pH 5 | 14 Tage |
| 17,18 | 200 mM Weinsäure pH 5 | 2 Tage |
| 19,20 | 200 mM Weinsäure pH 5 | 6 Tage |
| 21,22 | 200 mM Weinsäure pH 5 | 9 Tage |
| 23,24 | 200 mM Weinsäure pH 5 | 14 Tage |

Die Spuren 25 und 26 zeigen eine Gesamt-RNA, die ohne vorherige Lagerung der Macrophagen mit Hilfe eines kommerziell erhältlichen Isolierungskits - wie z. B. RNeasy^{®} Mini Kits der Firma QIAGEN - aus je 1 x 10⁶ Macrophagen isoliert wird (= Positiv-Kontrolle).

Die Menge an isolierter Gesamt-RNA wird nach Verdünnung in Wasser durch photometrische Messung der Lichtabsorption bei einer Wellenlänge von 260 nm ermittelt. Die Reinheit der so gewonnenen RNA wird durch die photometrische Bestimmung des Verhältnisses der Lichtabsorption bei 260 nm zu derjenigen bei 280 nm bestimmt. Die Ergebnisse der Isolierungen sind in der nachfolgenden Tabelle 10 dargestellt. Es werden jeweils die Mittelwerte der Doppelbestimmung angegeben.

**Tabelle 10: Nukleinsäure-Ausbeute aus den nach Beispiel 12 in 4% Tetradecyltrimethylammonium=Oxalat, 200 mM Additiv gelagerten Macrophagen**

| Additiv | Lagerung | RNA-Ausbeute (µg) | E₂₆₀/E₂₈₀ |
|---|---|---|---|
| 200 mM Phosphorsäure | 2 Tage | 24,2 | 1,91 |
| pH 5 | | | |
| 200 mM Phosphorsäure | 6 Tage | 25,7 | 1,86 |
| pH 5 | | | |
| 200 mM Phosphorsäure | 9 Tage | 21,6 | 1,83 |
| pH 5 | | | |
| 200 mM Phosphorsäure | 14 Tage | 23,5 | 1,83 |
| pH 5 | | | |
| 200 mM Tartronsäure pH 5 | 2 Tage | 241 | 1,86 |
| 200 mM Tartronsäure pH 5 | 6 Tage | 23,2 | 1,85 |
| 200 mM Tartronsäure pH 5 | 9 Tage | 20,2 | 1,86 |
| 200 mM Tartronsäure pH 5 | 14 Tage | 27,8 | 1,81 |
| 200 mM Weinsäure pH 5 | 2 Tage | 25,4 | 1,85 |
| 200 mM Weinsäure pH 5 | 6 Tage | 30,9 | 1,84 |
| 200 mM Weinsäure pH 5 | 9 Tage | 24,3 | 1,86 |
| 200 mM Weinsäure pH 5 | 14 Tage | 25,1 | 1,86 |
| Positiv-Kontrolle | ohne Lagerung | 16,3 | 1,88 |

Die gelelektrophoretische Auftrennung zeigt nach Anfärbung mit Ethidiumbromid die intakte 28S und 18S rRNA-Banden in den gelagerten ebenso wie in den nicht gelagerten Proben, wobei auch noch 14-tägier Lagerung kein RNA-Abbau zu erkennen ist. Ebenso bleiben die mittels photometrischer Messung bestimmten RNA-Ausbeuten und RNA-Reinheiten während der Lagerung unverändert.

### Beispiel 13:

### RNA-Stabilisierung in adhärenten Hela-Zellen ohne Entfernung des Mediums

Diese Experimente zeigen, dass mittels Tetradecyltrimethylammonium-Oxalat-Additiv-Mischungen RNA auch in adhärenten Zellen stabilisiert werden kann. Die Stabilisierung erfolgt dabei auch, wenn das Medium in dem sich die Zellen befinden, nicht entfernt, sondern die Tetradecyltrimethylammonium-Oxalat/Additiv-Mischung zum Medium hinzu gegeben wird. Zellen im Medium können dabei als Modell für Zellen in Körperflüssigkeiten angesehen werden.

Zur Herstellung der in diesem Experiment verwendeten Lösungen werden Tetradecyltrimethylammonium-Oxalat und das jeweilige Additiv Weinsäure bzw. Ammoniumsulfat für eine Endkonzentration von 4% Tetradecyltrimethylammonium-Oxalat und 200 mM Additiv eingewogen und in Wasser gelöst. Der pH-Wert der Lösung wird im Falle von 4% Tetradecyltrimethylammonium-Oxalat, 200 mM Weinsäure mit Natronlauge und im Falle von 4% Tetradecyltrimethylammonium-Oxalat, 200 mM Ammoniumsulfat mit Schwefelsäure auf pH 5 eingestellt. Hela-Zellen werden in 6-Well Plates in je 2 ml Medium angezogen. Die Zellen wachsen adhärent, d. h.: sie haften auf dem Boden des Wells. Zur RNA-Stabilisierung in den Zellen werden zu je einem well jeweils 10 ml 4% Tetradecyltrimethylammonium-Oxalat, 200 mM Weinsäure pH 5 bzw. 4% Tetradecyltrimethylammonium-Oxalat, 200 mM Ammoniumsulfat pH 5 zugegeben und die Platten für 4 Tage bei RT gelagert. Als Negativ-Kontrolle wird ein well mit Medium aber ohne Zugabe einer 4% Tetradecyltrimethylammonium-Oxalat, 200 mM Additiv-Mischung für 4 Tage bei RT gelagert.

Als Positiv-Kontrolle wird von einem well die RNA der Hela-Zellen ohne vorherige Lagerung mit Hilfe eines handelsüblichen Isolierungskits - wie z. B. RNeasy^{®} Mini Kits der Firma QIAGEN - isoliert. Hierfür wird das Medium vollständig von den Zellen abgenommen und mit 350 µl des Lysepuffers RLT (Bestandteil des RNeasy-Kits) versetzt. Die Zellen werden mit einem Schaber vom Boden des Wells abgeschabt und das Lysat in einen sog. Shredder - wie z. B. der QlAshredder der Firma QIAGEN - überführt. Durch Zentrifugation für 2 min bei 14000 rpm wird das Lysat durch den Shredder geführt und so die Probe homogenisiert. Der Durchfluß wird mit 70% Ethanol vermischt und wie in Beispiel 10 beschrieben, wird die RNA isoliert.

Nach 4 Tagen Lagerung der Zellen in einem Medium gemischt mit 4% Tetradecyltrimethylammonium-Oxalat, 200 mM Additiv werden die nun abgelösten Zellen vollständig zusammen mit dem Überstand aufgenommen und 5 min bei 3000xg zentrifugiert. Die Überstände werden abgenommen und das Zellpellet zur RNA-Isolierung, wie in Beispiel 10, beschrieben verwendet.

Nach 4 Tagen Lagerung der Zellen in einem Medium ohne 4% Tetradecyltrimethylammonium-Oxalat, 200 mM Additiv (= Negativ-Kontrolle) wird die RNA wie oben für die Positiv-Kontrolle beschrieben isoliert.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 20 µl des Eluates eingesetzt. Das Ergebnis ist in Fig. 13 wiedergegeben. Spur 1 enthält Gesamt-RNA, die nach Lagerung der Zellen in Medium gemischt mit 4% Tetradecyltrimethylammonium-Oxalat, 200 mM Weinsäure, pH 5 isoliert wird. Spur 2 zeigt Gesamt-RNA, die nach Lagerung der Zellen in Medium gemischt mit 4% Tetradecyltrimethylammonium-Oxalat, 200 mM Ammoniumphosphat, bei pH 5 isoliert wird. Die Spur 3 zeigt eine Gesamt-RNA, die nach Lagerung der Zellen nur im Medium isoliert wird und die Spur 4 zeigt eine Gesamt-RNA, die als Positiv-Kontrolle ohne vorherige Lagerung isoliert wird.

Die Menge an isolierter Gesamt-RNA wird nach Verdünnung in Wasser durch photometrische Messung der Lichtabsorption bei einer Wellenlänge von 260 nm ermittelt. Die Reinheit der so gewonnenen RNA wird durch die photometrische Bestimmung des Verhältnisses der Lichtabsorption bei 260 nm zu derjenigen bei 280 nm bestimmt. Die Ergebnisse der Isolierungen sind in der nachfolgenden Tabelle 11 dargestellt.

**Tabelle 11: RNA-Ausbeute der nach Beispiel 13 aus adhärenten Hela-Zellen isolierten Gesamt-RNA.**

| Lagerung in Medium gemischt mit | RNA-Ausbeute (µg) | E₂₆₀/E₂₈₀ |
|---|---|---|
| 4% Tetradecyltrimethylammonium-Oxalat, 200 mM Weinsäure, pH 5 | 10,9 | 1,70 |
| 4% Tetradecyltrimethylammonium-Oxalat, 200 mM Ammoniumsulfat, pH 5 | 13,2 | 1,75 |
| - | 6,1 | 1,58 |
| Positiv-Kontrolle ohne Lagerung | 12,5 | 1,75 |

Die gelelektrophoretische Auftrennung zeigt nach Anfärbung mit Ethidiumbromid die intakte 28S und 18S rRNA-Banden in der nicht gelagerten Probe ebenso wie in den mit Tetradecyltrimethylammonium-Oxalat-Additiv-Mischungen gelagerten Proben. Dagegen ist die RNA in den Zellen, die in Medium ohne Tetradecyltrimethylammonium-Oxalat-Additiv-Zugabe gelagert werden, nahezu vollständig abgebaut. Ebenso besteht kein Unterschied zwischen nicht-gelagerten und stabilisierten Proben bezüglich der mittels OD-Messung bestimmten RNA-Ausbeute und -Reinheit, während die Ausbeute und Reinheit der RNA in den in Medium ohne Tetradecyltrimethylammonium-Oxalat-Additiv-Zugabe gelagerten Proben deutlich reduziert ist.

### Beispiel 14:

### RNA-Stabilisierung in Gewebe mittels Tetradecyltrimethylammonium-Oxalat gemischt mit verschiedenen Additiven

Diese Experimente zeigen, dass Tetradecyltrimethylammonium-Oxalat gemischt mit verschiedenen Additiven auch geeignet ist RNA aus Gewebe zu stabilisieren.

Zur Herstellung der in diesem Experiment verwendeten Lösungen wird eine Stammlösung von 20% Tetradecyltrimethylammonium-Oxalat mit jeweils einer Stammlösung von 0,5 M von Weinsäure, Zitronensäure, Tartronsäure, Ammoniumsulfat, Kaliumphosphat, Oxalsäure oder Phosphorsäure zu einer Endkonzentration von 4% Tetradecyltrimethylammonium-Oxalat und 200 mM des Additivs vermischt. Die Stammlösungen der Additive werden vor der Mischung mit Tetradecyltrimethylammonium-Oxalat mittels Natronlauge oder Schwefelsäure (oder Ammoniumsulfat) oder Kalilauge bzw. Phosphorsäure (oder Kaliumphosphat) auf den jeweils angegebenen pH-Wert eingestellt.

Nierengewebe der Maus, welches nach Entnahme unverzüglich in flüssigem Stickstoff eingefroren und anschließend bei -70°C gelagert wurde, wird für diese Experimente verwendet. Je ca. 70 bis 90 mg des Gewebes werden gefroren mit 500 µl je 10 mg Gewebe der in Tabelle 12 genannten Puffer versetzt und sofort mittels eines Rotor-Stator-Homogenisators - wie z. B. des Polytrons der Firma Kinematica - für 30 bis 60 s homogenisiert. Von diesen Homogenisaten werden Aliquots von je 500 µl Lösung abgenommen, die somit 10 mg Gewebe entsprechen. Die Proben werden für einen Tag bei RT gelagert.

Im Anschluß an die Lagerung werden die Proben für 3 min bei 10000 xg zentrifugiert und der Überstand abgenommen. Das Pellet wird in 600 µl eines handelsüblichen Guanidinium-Isothiocyanat-Puffers - wie z. B. RLT-Puffer der Firma QIAGEN - durch Vortexen vollständig gelöst. Anschließend wird 1 Volumen (600 µl) 70 %-iges Ethanol zugefügt und durch mehrmaliges Auf- und Abpipettieren oder durch Vortexen über einen Zeitraum von ca. 5 s gemischt. Das Lysat wird anschließend in eine handelsübliche Silicamembran enthaltene Spin-Säule - wie z. B. RNeasy-Säulen der Firma QIAGEN - aufgetragen und durch Zentrifugation (1 min bei 10000 xg) durch die Membran hindurchgeführt. Die RNA bleibt an der Membran gebunden und wird anschließend wird mit einem ersten handelsüblichen Guanidinium-Isothiocyanat-haltigen Waschpuffer, - beispielsweise mit dem Puffer RW1 der Firma QIAGEN - und danach mit einem zweiten alkoholhaltigen Waschpuffer - z. B. dem Puffer RPE der Firma QIAGEN - gewaschen. Dabei werden die Waschpuffer jeweils durch Zentrifugation (1 min bei 10000 xg) durch die Membran hindurchgeführt. Die Waschung mit dem zweiten alkoholhaltigen Waschpuffer wird mit einem geringeren Volumen wiederholt wobei gleichzeitig die Membran durch die Zentrifugation (2 min max. rpm, hier 20000xg) getrocknet wird. Zur Elution werden 40 µl RNase-freies Wasser auf die Membran pipettiert, um die gereinigte RNA von der Membran abzulösen. Durch Zentrifugation (1 min bei 10000 xg) wird das Eluat durch die Membran hindurchgeführt und der Elutionsschritt wird zum Zwecke einer vollständigen Elution noch einmal wiederholt.

Die isolierte RNA wird auf Agarosegelen, die mit Ethidiumbromid angefärbt sind, analysiert. Hierzu werden beispielsweise 1,0 %-ige Formaldehyd-Agarose-MOPS-Gele angefertigt. Es werden jeweils 20 µl des Eluates eingesetzt. Das Ergebnis ist in Fig. 14 wiedergegeben. Die Proben sind in Tabelle 12 zusammengefaßt, wobei alle Proben jeweils 2x durchgeführt und dargestellt worden sind.

**Tabelle 12: Zusammenfassung der in Fig. 14 dargestellten Proben**

| Proben Nr. | Additiv | pH-Wert des Additivs | finaler pH-Wert der Mischung aus Tetradecyltrimethylammonium-Oxalat und Additiv |
|---|---|---|---|
| 1 | Weinsäure | 3 | 3,4 |
| 2 | Weinsäure | 4 | 4,3 |
| 3 | Weinsäure | 5 | 5,3 |
| 4 | Weinsäure | 6 | 6,0 |
| 5 | Weinsäure | 7 | 7,3 |
| 6 | Zitronensäure | 3 | 3,3 |
| 7 | Zitronensäure | 4 | 4,3 |
| 8 | Zitronensäure | 5 | 5,4 |
| 9 | Zitronensäure | 6 | 6,3 |
| 10 | Zitronensäure | 7 | 7,5 |
| 11 | Oxalsäure | 4 | 4,3 |
| 12 | Oxalsäure | 5 | 5,3 |
| 13 | Oxalsäure | 6,17 | 6,5 |
| 14 | Oxalsäure | 7 | 7,2 |
| 15 | Phosphorsäure | 3 | 4,3 |
| 16 | Phosphorsäure | 4 | 4,9 |
| 17 | Phosphorsäure | 5 | 6,0 |
| 18 | Phosphorsäure | 6 | 6,3 |
| 19 | Phosphorsäure | 7 | 7,1 |
| 20 | Kaliumphosphat | 4,2 | 4,9 |
| 21 | Kaliumphosphat | 5 | 5,3 |
| 22 | Kaliumphosphat | 6 | 6,1 |
| 23 | Kaliumphosphat | 7 | 6,9 |
| 24 | Kaliumphosphat | 8 | 7,8 |
| 25 | Tartronsäure | 3 | 3,6 |
| 26 | Tartronsäure | 4 | 4,4 |
| 27 | Tartronsäure | 5 | 5,3 |
| 28 | Tartronsäure | 6 | 5,9 |
| 29 | Tartronsäure | 7 | 7,3 |
| 30 | Ammoniumsulfat | 2 | 4,1 |
| 31 | Ammoniumsulfat | 3 | 5,2 |
| 32 | Ammoniumsulfat | 4 | 6,0 |
| 33 | Ammoniumsulfat | 5 | 6,1 |

Die Proben "K" zeigen eine Gesamt-RNA, die ohne vorherige Lagerung mit Hilfe eines Isolierungskits (RNeasy der Fa. Qiagen GmbH) aus 10 mg gefrorenem Nierengewebe isoliert wird (= Positiv-Kontrolle). Die Spuren "N" zeigen eine Gesamt-RNA, die nach eintägiger Lagerung von 10 mg Nierengewebe trocken, d. h. ohne Lösungszugabe mit Hilfe des RNeasy^{®} Mini Kits der Firma QIAGEN isoliert wird (= Negativ-Kontrolle.)

Die gelelektrophoretische Auftrennung der zeigt nach Anfärbung mit Ethidiumbromid die intakte 28S und 18S rRNA-Banden in der Positiv-Kontrolle. Die Negativ-Kontrolle, ohne Stabilisierungslösung gelagertes Nierengewebe, zeigt vollständig abgebaute RNA. Im Gegensatz dazu sind nach Lagerung der Proben in Tetradecyltrimethylammonium-Oxalat gemischt mit verschiedenen Additiven wie in der Positiv-Kontrolle die intakten rRNA-Banden sichtbar. Die Stabilisierung wird dabei vom pH-Wert beeinflußt. Bei der RNA-Stabilisierung in Gewebe werden finale pH-Werte der Stabilisierungslösung nach Mischung von Tetradecyltrimethylammonium-Oxalat und Additiv definierten pH-Wertes von größer 4 bevorzugt.

### Beispiel 15:

### DNA-Stabilisierung und -Isolierung parallel zur RNA-Stabilisierung und -Isolierung

Diese Experimente zeigen, dass mittels Tetradecyltrimethylammonium-Oxalat gemischt mit verschiedenen Additiven neben RNA auch DNA in Gewebe stabilisiert wird. Aus einer Probe kann dabei neben der RNA auch die DNA parallel isoliert werden.

Zur Herstellung der in diesem Experiment verwendeten Lösungen wird eine Stammlösung von 20% Tetradecyltrimethylammonium-Oxalat mit einer Stammlösung von 0,5 M von Zitronensäure pH 5, eingestellt mit Natronlauge, zu einer Endkonzentration von 4% Tetradecyltrimethylammonium-Oxalat und 200 mM des Additivs vermischt.

Nierengewebe der Maus, welches nach Entnahme unverzüglich in flüssigem Stickstoff eingefroren und anschließend bei -70°C gelagert wurde, wird für diese Experimente verwendet. Ca. 80 mg des Gewebes werden gefroren mit 4,2 ml 4% Tetradecyltrimethylammonium-Oxalat, 200 mM Zitronensäure pH 5 versetzt und sofort mittels eines Rotor-Stator-Homogenisators wie z. B. des Polytrons der Firma Kinematica für 30 bis 60 sek. homogenisiert. Von diesem Homogenisat werden Aliquots von je 500 µl Lösung abgenommen, die somit 10 mg Gewebe entsprechen. Die Proben werden für einen Tag bei RT gelagert.

Im Anschluß an die Lagerung werden die Proben für 3 min bei 10000 xg zentrifugiert und der Überstand abgenommen. Das Pellet wird in 600 µl eines handelsüblichen Guanidinium-Isothiocyanat-Puffers - wie z. B. RLT-Puffer der Firma QIAGEN - durch Vortexen vollständig gelöst. Anschließend wird 1 Volumen (600 µl) 70 %-iges Ethanol zugefügt und durch mehrmaliges Auf- und Abpipettieren oder durch Vortexen über einen Zeitraum von ca. 5 s gemischt. Das Lysat wird anschließend in eine handelsübliche Silicamembran enthaltene Spin-Säule - wie z. B. RNeasy-Säulen der Firma QIAGEN - aufgetragen und durch Zentrifugation (1 min bei 10000 xg) durch die Membran hindurchgeführt. Die RNA bleibt an der Membran gebunden und kann anschließend wie in Beispiel 14 beschrieben isoliert werden. Der Durchfluß (ca. 1200 µl) wird aufgefangen und mit 200 µl 100% Ethanol versetzt und durch Vortexen gemischt. Diese Proben werden erneut in eine handelsübliche Silicamembran enthaltene Spin-Säule - wie z. B. QlAamp-Säulen der Firma QIAGEN - aufgetragen und durch Zentrifugation (1 min bei 10000 xg) durch die Membran hindurchgeführt. Die DNA bleibt an der Membran gebunden und wird anschließend mit einem ersten handelsüblichen Guanidinium-lsothiocyanat-haltigen Waschpuffer - beispielsweise mit dem Puffer RW1 der Firma QIAGEN - und danach mit einem zweiten alkoholhaltigen Waschpuffer - z. B. Puffer RPE der Firma QIAGEN - gewaschen. Dabei werden die Waschpuffer jeweils durch Zentrifugation (1 min bei 10000 xg) durch die Membran geführt. Die Waschung mit dem zweiten alkoholhaltigen Waschpuffer wird mit einem geringeren Volumen wiederholt wobei gleichzeitig die Membran durch die Zentrifugation (2 min max. rpm, hier 20000xg) getrocknet wird. Zur Elution werden 200 µl Wasser auf die Membran pipettiert und 1 min bei RT inkubiert, um die gereinigte DNA von der Membran abzulösen. Durch Zentrifugation (1 min bei 10000 xg) wird das Eluat durch die Membran hindurchgeführt und der Elutionsschritt wird zum Zwecke einer vollständigen Elution noch einmal wiederholt.

Die isolierte DNA wird auf Agarosegelen, die mit Ethidiumbromid angefärbt sind, analysiert. Hierzu werden beispielsweise 0,8 %-ige Agarose-TBE-Gele angefertigt. Es werden jeweils 40 µl der Proben 1 bis 4 und 20 µl der Proben 5 bis 9 eingesetzt. Das Ergebnis ist in Fig. 15 wiedergegeben.

Die Spuren 1 und 2 zeigen die entsprechend Beispiel 15 isolierte Gesamt-DNA. Die Spuren 3 und 4 zeigen 0,1 µg bzw. 0,5 µg einer Gesamt-DNA als Referenz, zur Demonstration des Laufverhaltens einer intakten genomischen DNA im verwendeten Agarosegel. Die Spur 5 zeigt eine Gesamt-DNA, die ohne vorherige Lagerung mit Hilfe eines kommerziell erhältlichen Isolierungskits (QIAamp^{®} Mini Kits der Firma QIAGEN GmbH) aus 10 mg gefrorenem Nierengewebe der Ratte isoliert wird (= Positiv-Kontrolle). Als Negativ-Kontrolle diente Gesamt-DNA, die nach eintägiger Lagerung von 10 mg Nierengewebe trocken, d. h. ohne Lösungszugabe, oder in A. dest, mit Hilfe des QIAamp^{®} Mini Kits der Firma QIAGEN isoliert wird. Diese DNA ist in den Spuren 6 und 7 (Lagerung trocken) und in den Spuren 8 und 9 (Lagerung in A. dest.) gezeigt.

Die gelektrophoretische Auftrennung zeigt hochmolekulare, nicht degradierte DNA sowohl in den Spuren, welche die Referenz-DNA zeigen als auch in den Spuren, die die DNA der nicht-gelagerten Positiv-Kontrolle enthalten. Die Lagerung des Gewebes trocken oder in Wasser führt zu einem vollständigen Abbau der DNA. Dagegen bleibt sie in den entsprechend Beispiel 15 behandelten Proben intakt und wird während der Lagerung nicht degradiert. Mischungen aus Tetradecyltrimethylammonium-Oxalat mit Additiven sind somit geeignet auch DNA in biologischen Proben zu stabilisieren und erlauben zudem eine parallele Isolierung von RNA und DNA aus einer Probe.

## Patentansprüche

1. Verfahren zur Stabilisierung von Nukleinsäuren, **dadurch gekennzeichnet, dass** eine Nukleinsäure-haltige biologische Probe mit einer wässrigen Komposition umfassend eine kationische Verbindung der allgemeinen Formel
Y⁺R₁R₂R₃R₄X⁻
worin
Y Stickstoff oder Phosphor
R₁, R₂, R₃ und R₄ unabhängig voneinander einen unverzweigten oder verzweigten C₁-C₂₀-Alkylrest und/oder einen C₆-C₂₀-Arylrest sowie einen C₆-C₂₆-Aralkylrest und
X⁻ ein Anion einer anorganischen oder organischen, ein- oder mehrbasischen Säure
bedeuten können
und mindestens ein Protonendonor
miteinander in Kontakt gebracht werden.

2. Verfahren nach Anspruch 1, **dadurch gekennzeichnet, dass** R₁ einen höheren Alkylrest und R₂, R₃ und R₄ jeweils eine Methylgruppe bedeutet.

3. Verfahren nach Anspruch 2, **dadurch gekennzeichnet, dass** R₁ einen Alkylrest mit 12, 14 oder 16 Kohlenstoffatomen und R₂, R₃ und R₄ jeweils eine Methylgruppe bedeutet.

4. Verfahren nach einem der Ansprüche 1 bis 3, **dadurch gekennzeichnet, dass** das Anion X⁻ aus der Gruppe der Anionen von Halogenwasserstoffsäuren oder Anionen ein- oder zweibasischer organischer Säuren ausgewählt wird.

5. Verfahren nach Anspruch 4, **dadurch gekennzeichnet, dass** das Anion X⁻ aus Anionen der Gruppe Bromid, Chlorid, Phosphat, Sulfat, Formiat, Acetat, Propionat, Oxalat, Malonat, Succinat oder Citrat ausgewählt wird.

6. Verfahren nach einem der Ansprüche 1 bis 5, **dadurch gekennzeichnet, dass** der Protonendonor aus der Gruppe der gesättigten aliphatischen Monocarbonsäuren, der aliphatischen Alkenyl-carbonsäuren, der gesättigten und/oder ungesättigten aliphatischen C₂-C₆-Dicarbonsäurender aliphatischen Hydroxy-di- oder tricarbonsäuren, der ungesättigten Tricarbonsäuren, der aliphatischen Ketodicarbonsäuren, der Aminosäuren oder aus der Gruppe der Mineralsäuren oder deren Salze alleine oder in Kombination ausgewählt wird.

7. Verfahren nach Anspruch 6, **dadurch gekennzeichnet, dass** als gesättigte aliphatische Monocarbonsäure eine C₁-C₆-Alkyl-carbonsäure oder Mischungen dieser Säuren eingesetzt werden.

8. Verfahren nach Anspruch 6, **dadurch gekennzeichnet, dass** als aliphatische gesättigte Monocarbonsäuren Essigsäure, Propionsäure, n-Buttersäure, n-Valeriansäure, Isovaleriansäure, Ethyl-methyl-essigsäure (2-Methyl-buttersäure), 2,2-Dimethylpropionsäure (Pivalinsäure), n-Hexansäure, n-Octansäure, n-Decansäure bzw. n-Dodecansäure (Laurinsäure) oder Mischungen der genannten Säuren eingesetzt werden.

9. Verfahren nach Anspruch 6, **dadurch gekennzeichnet, dass** als aliphatische Alkenyl-carbonsäure Acrylsäure (Propensäure), Methacrylsäure, Crotonsäure, iso-Crotonsäure oder Vinylessigsäure oder Mischungen der genannten Säuren eingesetzt werden.

10. Verfahren nach Anspruch 6, **dadurch gekennzeichnet, dass** als gesättigte aliphatische C₂-C₆-Dicarbonsäure eine Dicarbonsäure aus der Gruppe Oxalsäure, Malonsäure, Bernsteinsäure, Glutarsäure bzw. Adipinsäure oder Mischungen der genannten Säuren eingesetzt werden.

11. Verfahren nach Anspruch 6, **dadurch gekennzeichnet, dass** als aliphatische Hydroxy-di- oder -tricarbonsäuren Tartronsäure, D-(+)-, L-(-)- oder DL-Äpfelsäure, (2R, 3R)-(+)-Weinsäure, (2S, 3S)-(-)-Weinsäure, meso-Weinsäure und Zitronensäure oder Mischungen der genannten Säuren eingesetzt werden.

12. Verfahren nach Anspruch 6, **dadurch gekennzeichnet, dass** als ungesättigte Dicarbonsäuren Malein- und/oder Fumarsäure oder Mischungen der genannten Säuren eingesetzt werden.

13. Verfahren nach Anspruch 6, **dadurch gekennzeichnet, dass** als ungesättigte Tricarbonsäure Aconitsäure eingesetzt wird.

14. Verfahren nach Anspruch 6, **dadurch gekennzeichnet, dass** als aliphatische Ketodicarbonsäuren Mesoxalsäure oder Oxalessigsäure, oder Mischungen der genannten Säuren eingesetzt werden.

15. Verfahren nach Anspruch 6, **dadurch gekennzeichnet, dass** als Aminosäuren Aminoessigsäure (Glycin), α-Aminopropionsäure (Alanin), α-Amino-iso-valeriansäure (Valin), α-Amino-iso*-*capronsäure (Leucin) und α-Amino-βmethylvaleriansäure (Isoleucin) oder Mischungen der genannten Säuren eingesetzt werden.

16. Verfahren nach einem der Ansprüche 1 bis 15, **dadurch gekennzeichnet, dass** die kationische Verbindung in einer Konzentration in einem Intervall von 0,01 Gew.-% bis zur Sättigung vorliegt.

17. Verfahren nach einem der vorhergehenden Ansprüche, **dadurch gekennzeichnet, dass** die Mischung aus der Nukleinsäure-haltigen biologischen Probe und der Komposition gegebenenfalls weitere Hilfsstoffe aufweist.

18. Verfahren nach einem der vorhergehenden Ansprüche, **dadurch gekennzeichnet, dass** als Nukleinsäuren Ribonukleinsäuren (RNA), Desoxyribonukleinsäuren (DNA) stabilisiert werden.

19. Verfahren nach Anspruch 17, **dadurch gekennzeichnet, dass** der pH-Wert der Mischung aus biologischer Probe und Komposition in einem Bereich von 2 bis 12 liegt.

20. Verfahren nach einem der vorhergehenden Ansprüche, **dadurch gekennzeichnet, dass** vor dem Mischen der biologischen Probe mit der Komposition oder in der Stabilisierungslösung die biologische Probe einer mechanischen, physikalischen, chemischen oder enzymatischen Einwirkung unterzogen wird.

21. Verfahren nach einem der vorhergehenden Ansprüche, **dadurch gekennzeichnet, dass** das Probenmaterial in der Komposition gelagert und anschließend direkt einer Nukleinsäure-Analytik oder -Detektion unterworfen wird.

22. Verfahren nach einem der vorhergehenden Ansprüche, **dadurch gekennzeichnet, dass** das Probenmaterial in der Komposition gelagert wird und anschließend die Nukleinsäuren aufgereinigt werden.

23. Verfahren nach Anspruch 22, **dadurch gekennzeichnet, dass** die Nukleinsäuren oder Nukleinsäure-haltigen Zellen oder Partikel, durch Filtration oder Zentrifugation von der restlichen Lösung abgetrennt werden und die abgetrennten Nukleinsäurensäuren oder Nukleinsäure-haltigen Zellen oder Partikel einer weiteren Aufreinigung unterworfen werden.

24. Verfahren nach einem der Ansprüche 1 bis 23, **dadurch gekennzeichnet, dass** die biologische Probe in der Mischung, die ggf. Viren oder Bakterien enthalten kann, Blut, Plasma oder Serum ist.

25. Verfahren nach Anspruch 24, **dadurch gekennzeichnet, dass** der pH-Wert der Mischung in einem Bereich von 2 bis 6 liegt.

26. Verfahren nach einem der Ansprüche 24 bis 25, **dadurch gekennzeichnet, dass** die Komplexe aus kationischer Verbindung und DNA und/oder RNA isoliert werden.

27. Verfahren nach Anspruch 26, **dadurch gekennzeichnet, dass** das bei der Isolierung erhaltene Pellet aus RNA bzw. DNA und kationischer Verbindung in einem Puffer aufgenommen wird.

28. Verfahren nach einem der Ansprüche 1 bis 23, **dadurch gekennzeichnet, dass** die biologische Probe, die ggf. Viren oder Bakterien enthalten kann, durch ein Punktat, Zellen, Gewebe oder Bakterien verkörpert wird.

29. Verfahren nach Anspruch 28, **dadurch gekennzeichnet, dass** der pH-Wert der Mischung in einem Bereich von 3 bis 10 liegt.

## Claims

1. Method for stabilizing nucleic acids, **characterized in that** a nucleic acid-containing biological sample, an aqueous composition comprising a cationic compound having the general formula
Y⁺R₁R₂R₃R₄X⁻
and at least one proton donor are brought into contact with each other, wherein,
Y may denote nitrogen or phosphorus;
R₁, R₂, R₃ and R₄ independent from each other may denote a un-branched or branched C₁-C₂₀-alkyl radical and/or a C₆-C₂₀-aryl radical as well as a C₆-C₂₆-aralkyl radical; and
X⁻ may denote an anion of an inorganic or organic, mono- or polybasic acid.

2. Method according to claim 1, **characterized in that** R¹ denotes a higher alkyl radical and R², R³ and R⁴, each denote a methyl group.

3. Method according to claim 2, **characterized in that** R¹ denotes a alkyl radical with 12, 14 or 16 carbon atoms and R², R³, and R⁴, each denote a methyl group.

4. Method according to one of claims 1 to 3, **characterized in that** the anion X⁻ is selected from the group of anions from hydrohalic acids or anions from mono- or di-basic organic acids.

5. Method according to claim 4, **characterized in that** the anion X⁻ is selected from anions of the group bromide, chloride, phosphate, sulphate, formate, acetate, propionate, oxalate, malonate, succinate or citrate.

6. Method according to one of claims 1 to 5, **characterized in that** the proton donor is selected alone or in combination from the group of saturated aliphatic mono-carboxylic acids, aliphatic alkenyl-carboxylic acids, saturated and/or unsaturated aliphatic C₂-C₆-di-carboxylic acids, aliphatic hydroxy di- or tri-carboxylic acids, unsaturated tri-carboxylic acids, aliphatic keto-di-carboxylic acids, amino acids or from the group of mineral acids or their salts.

7. Method according to claim 6, **characterized in that** as saturated aliphatic mono-carboxylic acid, a C₁-C₆-alkyl-carboxylic acid or mixtures of these acids are used.

8. Method according to claim 6, **characterized in that** as aliphatic saturated mono-carboxylic acids, acetic acid, propionic acid, n-butyric acid, n-valeric acid, isovaleric acid, ethyl-methyl-acetic acid (2-methyl-butyric acid), 2,2-di-methylpropionic acid (pivalic acid), n-hexanoic acid, n-octanoic acid, n-decanoic acid or n-dodecanoic acid (lauric acid) respectively or mixtures of said acids are used.

9. Method according to claim 6, **characterized in that** as aliphatic alkenyl-carboxylic acid, acrylic acid (propenoic acid), methacrylic acid, crotonic acid, iso-crotonic acid or vinylacetic acid or mixtures of said acids are used.

10. Method according to claim 6, **characterized in that** as aliphatic saturated C₂-C₆-di-carboxylic acid, a di-carboxylic acid from the group of oxalic acid, malonic acid, succinic acid, glutaric acid or adipic acid respectively or mixtures of said acids are used.

11. Method according to claim 6, **characterized in that** as aliphatic hydroxy di- or tri-carboxylic acids, tartronic acid, D-(+), L-(-) or DL-malic acid, (2R, 3R)-(+)-tartaric acid, (2S, 3S)-(-)-tartaric acid, meso-tartaric acid and citric acid or mixtures of said acids are used.

12. Method according to claim 6, **characterized in that** as unsaturated di-carboxylic acids, maleic- and/or fumaric acid or mixtures of said acids are used.

13. Method according to claim 6, **characterized in that** as unsaturated tri-carboxylic acid, aconitic acid is used.

14. Method according to claim 6, **characterized in that** as aliphatic keto-di-carboxylic acids, mesoxalic acid or oxaloacetic acid, or mixtures of said acids are used.

15. Method according to claim 6, **characterized in that** as amino acids, aminoacetic acid (glycine), α-aminopropionic acid (alanine), α-amino-iso-valeric acid (valine), α-amino-iso-caproic acid (leucine), and α-amino-β-methylvaleric acid (isoleucine) or mixtures of said acids are used.

16. Method according to one of claims 1 to 15, **characterized in that** the cationic compound is present in a concentration from one interval of 0,01 % by weight up to saturation.

17. Method according to one of the preceding claims, **characterized in that** the mixture of the nucleic acid-containing biological sample and the composition, optionally, contains further additives.

18. Method according to one of the preceding claims, **characterized in that** as nucleic acids, ribonucleic acids (RNA), deoxyribonucleic acids (DNA) are stabilized.

19. Method according to claim 17, **characterized in that** the pH-value of the mixture of biological sample and composition lies in a range from 2 to 12.

20. Method according to one of the preceding claims, **characterized in that** before mixing the biological sample with the composition or in the stabilizing solution, the biological sample is subjected to a mechanical, physical, chemical or enzymatic action.

21. Method according to one of the preceding claims, **characterized in that** the sample material is stored in the composition and subsequently directly subjected to nucleic acid-analytics or -detection.

22. Method according to one of the preceding claims, **characterized in that** the sample material is stored in the composition and subsequently the nucleic acids are purified.

23. Method according to claim 22, **characterized in that** the nucleic acids or nucleic acid-containing cells or particles are separated from the remaining solution by filtration or centrifugation and the separated nucleic acids or nucleic acid-containing cells or particles are subjected to a further purification.

24. Method according to one of claims 1 to 23, **characterized in that** the biological sample in the mixture, which optionally may contain viruses or bacteria, is blood, plasma or serum.

25. Method according to claim 24, **characterized in that** the pH-value of the mixture lies in a range of 2 to 6.

26. Method according to one of claims 24 to 25, **characterized in that** the complexes of cationic compounds and DNA and/or RNA are isolated.

27. Method according to one of claim 26, **characterized in that** the pellet obtained during isolation, containing RNA or DNA and cationic compound, is taken-up into a buffer.

28. Method according to one of claims 1 to 23, **characterized in that** the biological sample, which optionally may contain viruses or bacteria, is represented by a punctate, cells, tissue or bacteria.

29. Method according to claim 28, **characterized in that** the pH-value of the mixture lies in a range of 3 to 10.

## Revendications

1. Procédé de stabilisation d'acides nucléiques, **caractérisé en ce qu'**un échantillon biologique renfermant des acides nucléiques est amené au contact d'une composition aqueuse renfermant un composé cationique de formule générale
Y⁺R₁R₂R₃R₄X⁻
dans laquelle
Y peut représenter l'azote ou le phosphore,
R₁, R₂, R₃ et R₄ peuvent représenter, indépendamment l'un de l'autre, un reste alkyle en C₁-C₂₀ non substitué ou substitué et/ou un reste aryle en C₆-C₂₀ ainsi qu'un reste aralkyle en C₆-C₂₆, et
X⁻ est un anion d'un acide inorganique ou organique, mono- ou polybasique,
et d'au moins un donneur de protons.

2. Procédé selon la revendication 1, **caractérisé en ce que** R₁ représente un reste alkyle supérieur et R², R₃ et R₄ représentent chacun un groupe méthyle.

3. Procédé selon la revendication 2, **caractérisé en ce que** R₁ représente un reste alkyle ayant 12, 14 ou 16 atomes de carbone et R₂, R₃ et R₄ représentent chacun un groupe méthyle.

4. Procédé selon l'une des revendications 1 à 3, **caractérisé en ce que** l'anion X⁻ est choisi dans le groupe des anions d'acides halogénohydriques ou d'anions d'acides organiques mono- ou dibasiques.

5. Procédé selon la revendication 4, **caractérisé en ce que** l'anion X⁻ est choisi dans le groupe des anions bromure, chlorure, phosphate, sulfate, formiate, acétate, propionate, oxalate, malonate, succinate ou citrate.

6. Procédé selon l'une des revendications 1 à 5, **caractérisé en ce que** le donneur de protons est choisi dans le groupe des acides aliphatiques saturés monocarboxyliques, des acides aliphatiques alkényl-carboxyliques, des acides aliphatiques saturés et/ou insaturés dicarboxyliques en C₂-C₆, des acides aliphatiques hydroxy-di- ou tricarboxyliques, des acides tricarboxyliques insaturés, des acides aliphatiques cétodicarboxyliques, des acides aminés ou dans le groupe des acides minéraux ou de leurs sels, seuls ou en combinaison.

7. Procédé selon la revendication 6, **caractérisé en ce que**, comme acide aliphatique saturé monocarboxylique, on utilise un acide (alkyle en C₁-C₆)carboxylique ou un mélange de tels acides.

8. Procédé selon la revendication 6, **caractérisé en ce que**, comme acide aliphatique saturé monocarboxylique, on utilise l'acide acétique, l'acide propionique, l'acide n-butyrique, l'acide n-valérique, l'acide isovalérique, l'acide éthyl-méthyl-acétique (acide 2-méthyl-butyrique), l'acide 2,2-diméthylpropionique (acide pivalique), l'acide n-hexanoïque, l'acide n-octanoïque, l'acide n-décanoïque, selon le cas, l'acide n-dodécanoïque (acide laurique) ou des mélanges des acides précités.

9. Procédé selon la revendication 6, **caractérisé en ce que**, comme acide aliphatique alkénylcarboxylique, on utilise l'acide acrylique (acide propénoïque), l'acide méthacrylique, l'acide crotonique, l'acide isocrotonique ou vinylacétique, ou des mélanges des acides précités.

10. Procédé selon la revendication 6, **caractérisé en ce que**, comme acide aliphatique saturé dicarboxylique en C₂-C₆, on utilise un acide dicarboxylique appartenant au groupe de l'acide oxalique, de l'acide malonique, de l'acide succinique, de l'acide glutarique, selon le cas, de l'acide adipinique, ou des mélanges des acides précités.

11. Procédé selon la revendication 6, **caractérisé en ce que**, comme acide aliphatique hydroxy-di- ou tricarboxylique, on utilise l'acide tartronique, l'acide D-(+) -, (L) - (-) - ou DL-malique, l'acide (2R,3R)-(+)-tartrique, l'acide (2S,3S)-(+)-tartrique, l'acide méso-tartrique et l'acide citrique, ou des mélanges des acides précités.

12. Procédé selon la revendication 6, **caractérisé en ce que**, comme acide dicarboxylique insaturé, on utilise l'acide maléique et/ou l'acide fumarique, ou des mélanges des acides précités.

13. Procédé selon la revendication 6, **caractérisé en ce que**, comme acide tricarboxylique insaturé, on utilise l'acide aconitique.

14. Procédé selon la revendication 6, **caractérisé en ce que**, comme acide aliphatique cétocarboxylique, on utilise l'acide méso-oxalique ou l'acide oxalo-acétique, ou des mélanges des acides précités.

15. Procédé selon la revendication 6, **caractérisé en ce que**, comme acide aminé, on utilise l'acide aminoacétique (glycine), l'acide α-aminopropionique (alanine), l'acide α-amino-iso-valérique (valine), l'acide α-amino-iso-caproïque (leucine) et l'acide α-amino-β-méthylvalérique (isoleucine), ou des mélanges des acides précités.

16. Procédé selon l'une des revendications 1 à 15, **caractérisé en ce que** le composé cationique est à une concentration comprise dans la gamme allant de 0,01 % en poids à la saturation.

17. Procédé selon l'une des revendications précédentes, **caractérisé en ce que** le mélange formé de l'échantillon biologique renfermant des acides nucléiques et de la composition présente, le cas échéant, d'autres excipients.

18. Procédé selon l'une des revendications précédentes, **caractérisé en ce que**, comme acide nucléique, sont stabilisés des acides ribonucléiques (ARN) et des acides désoxyribonucléiques (ADN).

19. Procédé selon la revendication 17, **caractérisé en ce que** la valeur du pH du mélange formé de l'échantillon biologique et de la composition est située dans la gamme allant de 2 à 12.

20. Procédé selon l'une des revendications précédentes, **caractérisé en ce qu'**il est exercé une action mécanique, physique, chimique ou enzymatique avant le mélange de l'échantillon biologique avec la composition ou dans la solution de stabilisation de l'échantillon biologique.

21. Procédé selon l'une des revendications précédentes, **caractérisé en ce que** le matériau de l'échantillon est conservé dans la composition et finalement directement soumis à une analyse ou détection d'acides nucléiques.

22. Procédé selon l'une des revendications précédentes, **caractérisé en ce que** le matériau de l'échantillon est conservé dans la composition et les acides nucléiques sont finalement purifiés.

23. Procédé selon la revendication 22, **caractérisé en ce que** les acides nucléiques ou les cellules ou particules contenant des acides nucléiques sont séparés du reste de la solution par filtration ou centrifugation et les acides nucléiques ou les cellules ou particules contenant des acides nucléiques ainsi séparés sont soumis à une purification supplémentaire.

24. Procédé selon l'une des revendications 1 à 23, **caractérisé en ce que** l'échantillon biologique dans le mélange, qui peut contenir éventuellement des virus ou des bactéries, est du sang, du plasma ou du sérum.

25. Procédé selon la revendication 24, **caractérisé en ce que** la valeur du pH du mélange est située dans la gamme allant de 2 à 6.

26. Procédé selon l'une des revendications 24 ou 25, **caractérisé en ce que** l'on isole le complexe formé du composé cationique et de l'ADN et/ou de l'ARN.

27. Procédé selon la revendication 26, **caractérisé en ce que** le culot issu de l'isolement, formé d'ARN, selon le cas d'ADN, et du composé cationique, est repris dans un tampon.

28. Procédé selon l'une des revendications 1 à 23, **caractérisé en ce que** l'échantillon biologique qui peut contenir éventuellement des virus ou des bactéries, revêt la forme d'une ponction, de cellules, d'un tissu ou de bactéries.

29. Procédé selon la revendication 28, **caractérisé en ce que** la valeur du pH du mélange est située dans la gamme allant de 3 à 10.
